# EUROPEAN PATENT SPECIFICATION

(11) **EP 4 189 063 B1**
(45) Date of publication and mention of the grant of the patent: **28.10.2026**
(21) Application number: 21850111.2
(22) Date of filing: 27.07.2021
(51) Int. Cl.: A61K 35/741, A61P 1/00, A61P 31/04

(54) **FORMULATION CONSISTING OF BACTERIAL STRAINS AND USE IN TREATING CLOSTRIDIOIDES DIFFICILE INFECTION**
FORMULIERUNG BESTEHEND AUS BAKTERIENSTÄMMEN UND ANWENDUNG ZUR BEHANDLUNG VON CLOSTRIDIOIDES-DIFFICILE-INFEKTIONEN
FORMULATION CONSTITUÉE DE SOUCHES BACTÉRIENNES ET UTILISATION DANS LE TRAITEMENT DE L'INFECTION À CLOSTRIODES DIFFICILE

(30) Priority: 28.07.2020 US 202063057492 P; 25.08.2020 US 202063069931 P
(43) Date of publication of application: 07.06.2023
(73) Proprietor: Icahn School of Medicine at Mount Sinai, New York, NY 10029 (US)
(72) Inventor: FAITH, Jeremiah, New York, New York 10029 (US); AGGARWALA, Varun, New York, New York 10029 (US); BETHLEHEM, Lukas, New York, New York 10029 (US); EGGERS, Joseph Jerome, New York, New York 10029 (US)
(74) Representative: Betten & Resch
(86) International application number: PCT/US2021/071018
(87) International publication number: WO 2022/027040

(56) References cited:
- WO-A1-2012/142605
- WO-A1-2013/037068
- WO-A2-2019/118515
- US-A1- 2018 353 554
- US-B1- 9 011 834
- US-B2- 10 064 904
- AGGARWALA VARUN ET AL: "Quantification of discrete gut bacterial strains following fecal transplantation for recurrent Clostridioides difficile infection demonstrates long-term stable engraftment in non-relapsing recipients", BIORXIV, 10 September 2020 (2020-09-10), XP093141047, Retrieved from the Internet <URL:https://www.biorxiv.org/content/10.1101/2020.09.10.292136v1.full.pdf> [retrieved on 20240313], DOI: 10.1101/2020.09.10.292136
- "Supplementary material", 10 September 2020 (2020-09-10), Retrieved from the Internet <URL:https://www.biorxiv.org/content/biorxiv/early/2020/09/10/2020.09.10.292136/DC1/embed/media-1.xlsx?download=true> [retrieved on 24100313]

## Description

### BACKGROUND

The mammalian gastrointestinal (GI) tract harbors a diverse microbial community that is usually maintained in symbiotic balance. Interactions between microbes within the microbial populations, and between the microbes and the host, affect both the host and the internal microbial community. In some individuals, this symbiotic balance is disrupted. This state can lead to increased susceptibility to pathogens and the development of disease. One such disease, *Clostridioides difficile* infection (CDI) is the leading cause of health care associated diarrhea, with approximately a half million cases and 29,000 deaths in the United States. CDI is associated with antibiotic-induced dysbiosis, and treatment typically consists of terminating administration of the antibiotic followed by antimicrobial therapy.

Recurrent *Clostridioides difficile* infection (rCDI) describes a clinical condition where *Clostridioides difficile* bacterial infections recur in a single patient after treatment for the original infection. Fecal Microbiota Transplantation (FMT) has been widely used therapeutically for recurrent rCDI, since its superiority to vancomycin was demonstrated. (*See, e.g.,* Ooijevaar, R. E., et al., Annu. Rev. Med. 70, 335-351 (2019)). With no FDA-approved drug, FMT is currently largely used under enforcement discretion in the USA. Although thousands of FMTs have been conducted over the last decade, many questions remain about the efficacy of different FMT formulations and the reasons for the long-term success or failures of different formulations. Open questions include, for example, identifying which FMT donor strains engraft in recipients, whether any FMT strains last beyond days or months, identifying the proportion of donor, recipient and environmental strains that ultimately survive, and how these different factors affect relapse, if at all.

A significant impediment to answering the above questions is the ability to and need for obtaining strain level resolution of the microbiome of the human gut. Previous microbiome analyses utilized a level of resolution that was incapable of delineating bacterial strains within a particular species. *See, e.g.,* Knight, R. et al., Nat. Rev. Microbiol. 16, 410-422 (2018). Pure metagenomics approaches, meanwhile, require very deep sequencing to track strains via SNPs in marker genes, do not model the microbiota as a defined set of discrete strains, and primarily provide non-quantifiable inferences related to sharing of metagenome-assembled bacterial contigs or SNPs across FMT samples. *See, e.g.,* Olm, M. R. et al., Nat. Biotechnol., 1-10 (2021). A higher level of resolution is required to determine the efficacy of any FMT formulation and its ultimate impact on the host.

In addition, recent FDA advisories have documented adverse events associated with FMT and have raised safety concerns about using FMT formulations that contain whole stool material. Moreover, FMT formulations are undefined, contain hundreds of strains, and can include both beneficial and potentially harmful microbes (including antibiotic resistant strains). A goal in the field is to generate a defined cocktail of microbes with demonstrated safety and efficacy that can be used instead of FMT to treat conditions such as rCDI. Another goal is to achieve consistent strain level monitoring methodologies that can be used to track disease and treatment efficacy.
WO 2012/142605 A1 relates to pharmaceutical compositions and methods suitable for the treatment of intestinal disorders and chronic diseases associated with the presence of abnormal distribution of microflora in the gastrointestinal tract of a mammalian host.
US 10 064 904 B2 relates to compositions of purified bacterial strains, and methods for treating or pathogenic infections, such as Clostridium difficile infections.
US 2018/353554 A1 relates to therapeutic compositions containing combinations of bacteria, for the maintenance or restoration of a healthy microbiota in the gastrointestinal tract of a mammalian subject, and methods for use thereof.
US 9 011 834 B1 relates to therapeutic compositions containing non-pathogenic, germination-competent bacterial spores, for the prevention, control, and treatment of gastrointestinal diseases, disorders and conditions and for general nutritional health.
WO 2019/118515 A2 relates to compositions and methods for the suppression of multi-drug-resistant organisms.
Aggarwala Varun ET AL: "Quantification of discrete gut bacterial strains following fecal transplantation for recurrent Clostridioides difficile infection demonstrates long-term stable engraftment in non-relapsing recipients", bioRxiv, 10 September 2020 (2020-09-10), DOI: 10.1101/2020.09.10.292136, relates to engrafting discrete bacterial strains for use in Live Biotherapeutic Products (LBP).
WO 2013/037068 A1 relates to synthetic stool preparation and methods of use thereof for treating disorders associated with dysbiosis of the gastrointestinal tract, such as Clostridium difficile infection, including recurrent Clostridium difficile infection.

### SUMMARY OF THE DESCRIPTION

The invention is defined in the independent claims. The present disclosure provides compositions for use in treating *Clostridioides difficile* infections, including for treating recurrent CDI, in the form of a Live Biotherapeutic Product (LBP).

The LBP of the present disclosure contains a live, cultured bacterial composition for engraftment into human patients suffering from gastrointestinal disorders, particularly *Clostridioides difficile* infections.

The LBP of the present disclosure contains FMT donor strains: that have been isolated and purified; that engraft consistently into recipient gut microbiotas.

The LBP of the present disclosure includes: live bacterial strains that have been isolated, purified and cultured; that engraft consistently into recipients; and that are susceptible to treatment with multiple antibiotic classes.

The LBP of the present disclosure includes: live bacterial strains that have been isolated, purified and cultured; that engraft consistently into recipients; that are susceptible to treatment with multiple antibiotic classes; and where none of the strains is resistant to any of the last line of antibiotics.

The present disclosure provides a composition comprising a formulation of bacterial strains for treating diseases, disorders, or maladies of the human gastrointestinal tract, wherein the formulation comprises a mixture of isolated, cultured bacteria selected from the group consisting of: *Bacteroides ovatus; Bacteroides vulgatus; Bifidobacterium longum; Bacteroides uniformis; Bacteroides thetaiotaomicron; Ruminococcus obeum; Parabacteroides distasonis; Coprococcus comes; Bacteroides fragilis; Dorea longicatena; Parabacteroides merdae; Bacteroides cellulosilyticus* '; *Bifidobacterium pseudocatenulatum; Odoribacter splanchnicus; Ruminococcus torques; Bacteroides caccae; Alistipes putredinis; Alistipes onderdonkii; Eubacterium rectale; Collinsella aerofaciens; Blautia massiliensis; Bacteroides stercoris; Barnesiella intestinihominis; Alistipes senegalensis; Bifidobacterium adolescentis; Eggerthella lenta; Clostridium ramosum; Bifidobacterium bifidum; Clostridium leptum; Streptococcus parasanguinis; Eubacterium siraeum; Streptococcus salivarius; Roseburia faecis; Bacteroides intestinalis; Escherichia coli; Bacteroides clarus; Bacteroides xylanisolvens; Parabacteroides johnsonii; Anaerotruncus colihominis; Bacteroides massiliensis; and Alistipes shahii.*

The present disclosure also provides for the first-time a high throughput hybrid approach for identifying bacterial strains in the microbial genome of a subject. The method involves collecting comprehensive cultures of bacterial strains from FMT donors or recipients and tracking the composition of the cultures across metagenomic samples using computational analysis and comparing the genomic results to reference sequences of the cultured strains.

### BRIEF DESCRIPTION OF DRAWINGS

Fig. 1 A-D illustrates how the *Strainer* algorithm accurately detects bacterial strains from complex gut communities and outperforms SNP-inference based metagenomics approaches.
Fig. 2 A-E illustrates the FMT strain dynamics in recipients after a single dose of FMT and how they can last for up to 5 years.
Fig. 3 A-D illustrates how donor engraftment of certain strains independently explains rCDI FMT clinical outcomes and identifies bacterial strains for LBP.
Fig. 4 A-E illustrates the Strainer algorithm, process for implementing it, and extent of the cultured bacterial strain library developed using it.
Fig. 5 A-F illustrates FMT strain dynamics (donor, pre-FMT recipient and novel environmental strains) in recipients post-FMT.
Fig. 6 A-B illustrates the clinical implications of engraftment of donor strains in a representative recipient and identifies frequently engrafting bacterial species with potential for LBP.

### DETAILED DESCRIPTION

The present disclosure fulfills the abovementioned needs by identifying for the first time a Live Biotherapeutic Product (LBP), which includes a defined sample of bacterial strains that are effective in treating gut disorders and in generating a durable, long-term change to the recipient's microbiome following a single administration. The present disclosure also provides methods for treating rCDI patients by quantifying the efficacy and long-term stability of FMT and LBP strains engrafted into patients with rCDI and modifying patient treatment accordingly.

In the present description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the present subject matter.

References in the specification to "one embodiment", "an embodiment", "an example embodiment" or "some embodiments," etc. indicate that the embodiments described may include a particular feature, structure, or characteristic, but every embodiment may not necessarily include the particular feature, structure, or characteristic. Moreover, such phrases are not necessarily referring to the same embodiment.

### Definitions:

The term "Live Biotherapeutic Product" or "LBP" as used herein refers to a composition containing a defined population of isolated, purified, and cultured bacterial strains that are effective for treating disorders of the gastrointestinal tract, particularly *Clostridioides difficile* infections, including rCDI. The population of bacteria in the LBP are susceptible to at least two different classes of antibiotics and can be sensitively and precisely detected in the recipient.

The term *"Clostridioides difficile* infection" or "rCDI" as used herein, refers to a clinical situation where a patient is diagnosed with a *Clostridioides difficile* infection, which has been clinically identified by symptoms, usually diarrhea, and a positive assay result for C. difficile toxin or detection of a toxin-producing C. difficile strain. The term "recurrent *Clostridioides difficile* infection" or "rCDI" is defined by resolution of CDI symptoms while on appropriate CDI therapy, followed by reappearance of symptoms within two to eight weeks after treatment has been stopped.

Unless otherwise defined herein, scientific and technical terms used in connection with the present disclosure shall have the meanings that are commonly understood by those of ordinary skill in the art. The meaning and scope of the terms should be clear, however, in the event of any latent ambiguity, definitions provided herein take precedent over any dictionary or extrinsic definition. Further, unless otherwise required by context, singular terms shall include pluralities and plural terms shall include the singular.

As used herein, the terms "comprising" (and any form of comprising, such as "comprise," "comprises," and "comprised"), "having" (and any form of having, such as "have" and "has"), "including" (and any form of including, such as "includes" and "include"), or "containing" (and any form of containing, such as "contains" and "contain"), are inclusive or open-ended and do not exclude additional, unrecited elements or method steps.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

"Consisting essentially of," when used in the claims, shall have its ordinary meaning as used in the field of patent law.

The term "about" is used herein to mean within the typical ranges of tolerances in the art. For example, "about" can be understood as about 2 standard deviations from the mean. According to certain embodiments, when referring to a measurable value such as an amount and the like, "about" is meant to encompass variations of ±20%, ±10%, ±5%, ±1%, ±0.9%, ±0.8%, ±0.7%, ±0.6%, ±0.5%, ±0.4%, ±0.3%, ±0.2% or ±0.1% from the specified value as such variations are appropriate to perform the disclosed methods. When "about" is present before a series of numbers or a range, it is understood that "about" can modify each of the numbers in the series or range.

### EXAMPLES

The below examples provide specific embodiments. The specific embodiments show exemplary compositions that can be made according to the teachings contained herein. The specific embodiments also show methods for staging, treating, and tracking the progression of treatment for rCDI that can be accomplished using the teachings herein. The use of these specific examples, however, is not intended to be limiting

### Example 1: FMT samples and isolation of strains:

The inventors isolated and sequenced the largest collection of 2,987 bacterial isolates representing 1,008 unique strains (207 species) from 9 FMT healthy donors and 13 rCDI FMT recipients **(Table 1)**. Similar to previous analyses performed by the inventors, bacterial isolates with <96% whole genome similarity were defined as unique strains, otherwise they were considered as multiple isolates for the representative strain.

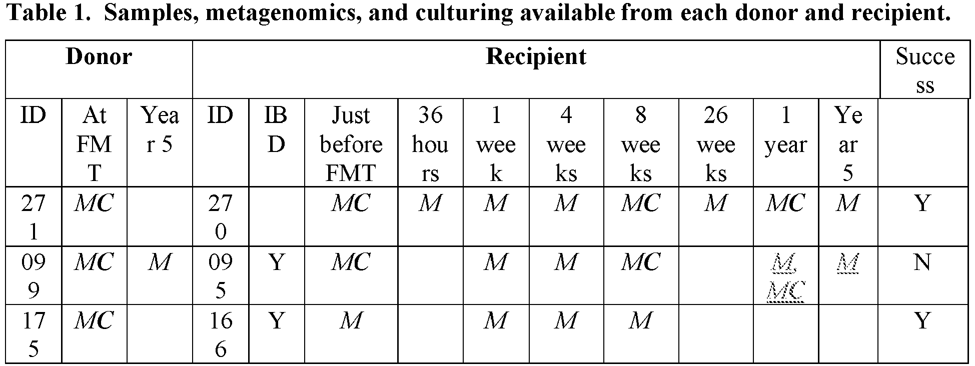

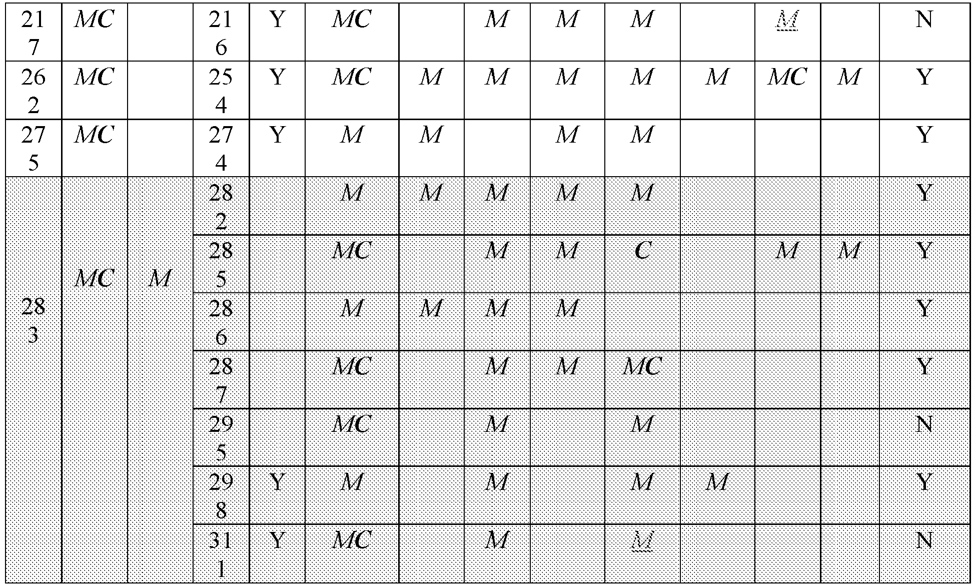

### Samples, metagenomics, and culturing available from each donor and recipient.

Seven donors provided their fecal material for FMT to 13 patients with either rCDI or both rCDI and IBD. Fecal metagenomics was performed on all stool samples. Donor strains from all the donors were isolated and tracked in matching recipient metagenomes over time. Strains were also isolated from a few recipients both pre- and post-FMT. *M* and *C* indicate that metagenomics or culturing respectively were performed at an indicated time point. The underline highlight denotes that a sample was collected after repeat FMT (due to initial failure of FMT). Success indicates that no relapse was noted for that patient.

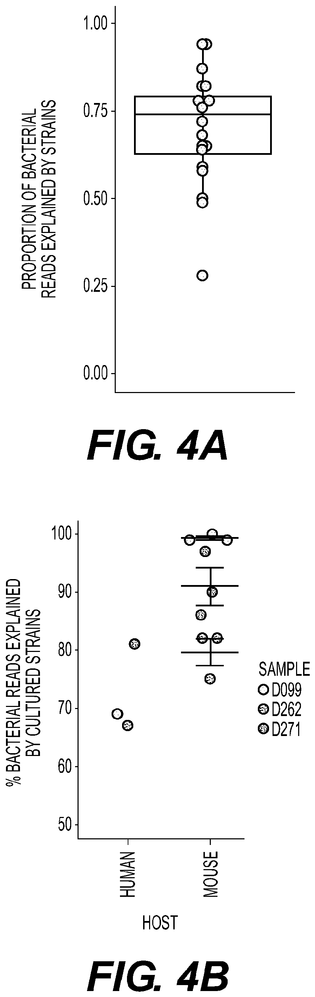

The inventors sequenced 85 metagenomes from donor fecal samples used for the transplant, and recipient samples taken prior to and for up to 5 years after FMT. The cultured strains represented the majority of the metagenome with 70% (sd = 16%) of bacterial metagenomic reads mapping to the cultured strain genomes (Figure 4A). The inventors also evaluated the comprehensiveness of the cultured bacterial strain library by gavaging several (n = 9) germ-free mice with human stool and performing metagenomics on the mouse fecal samples. The cultured bacterial strains now explained up to 90% (sd = 9.2%, Figure 4B) of bacterial reads in the gnotobiotic mice colonized with the relevant human stool, illustrating that the majority of unexplained bacterial metagenomics reads in the human sample were from unculturable sources (e.g. dead bacteria from food and environmental sources).

### Example 2: Strainer algorithm for tracking strains.

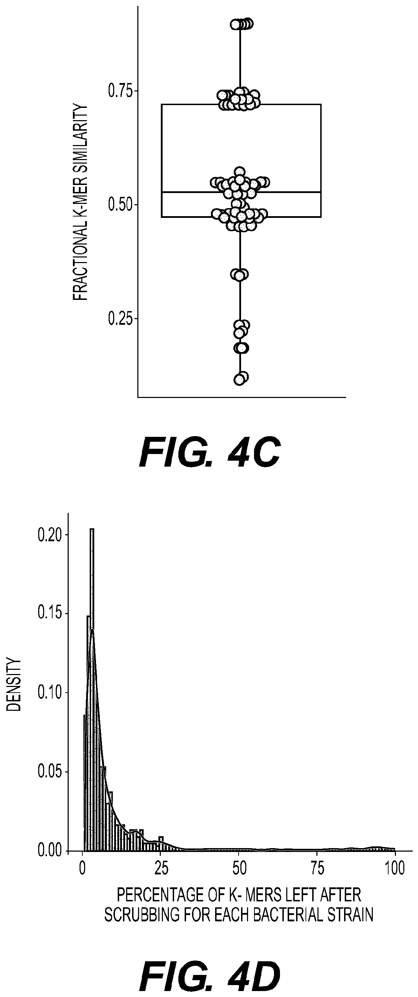

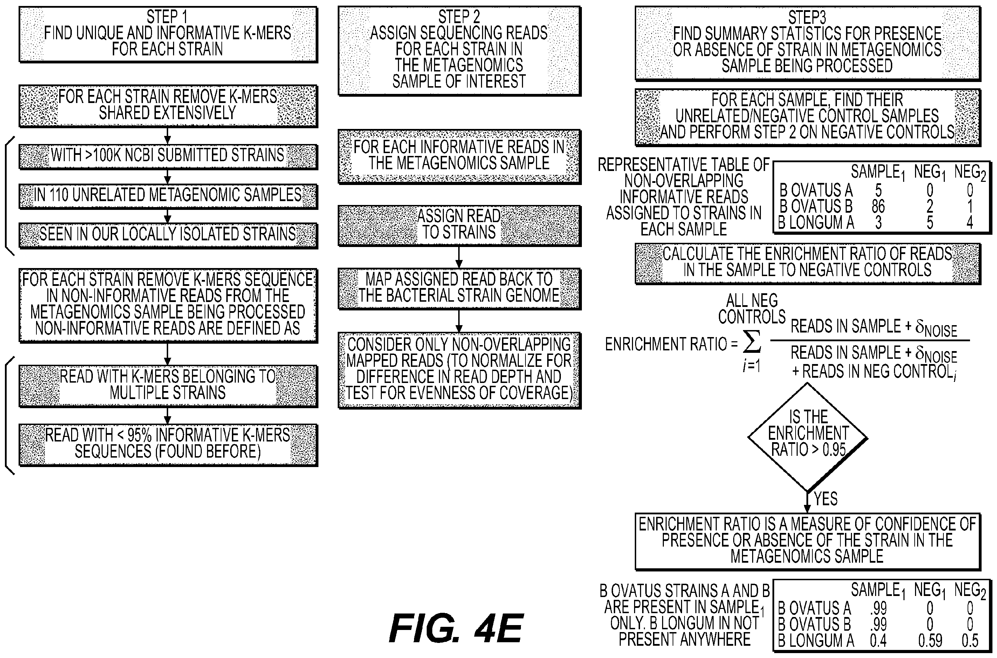

The present disclosure overcomes one of the central challenges behind strain tracking from metagenomics data, i.e., the identification of a set of informative sequence features or k-mers from the bacterial genome that can uniquely identify a given strain. Bacterial species often contain numerous closely related distinct strains that share a majority of their genomic content **(****Figure 4C****);** therefore, the identification of informative features that make it possible to track strains was a challenge that the present disclosure overcomes. The inventors were able to obtain the most informative k-mers (k = 31) for a strain only after removal of those shared extensively with bacterial genomes and fecal metagenomes from unrelated non-cohabitating individuals where the probability of occurrence of the same strain is very low **(Table 7,** **Figures 4D** and **4E****).** To achieve this task, the inventors assigned each sequencing read in a metagenomics sample to a unique strain by comparing the distribution of k-mers on a read with the informative k-mers identified earlier for that strain. Next, the inventors mapped these assigned reads for a strain to its genome, to adjust for sequencing depth and evenness of coverage. Finally, the inventors compared the number of positionally distinct reads for a strain in the metagenomic sample with those found in unrelated samples and assign a confidence score for presence of that strain.

### Example 3: Validation on a defined community of strains in gnotobiotic mice.

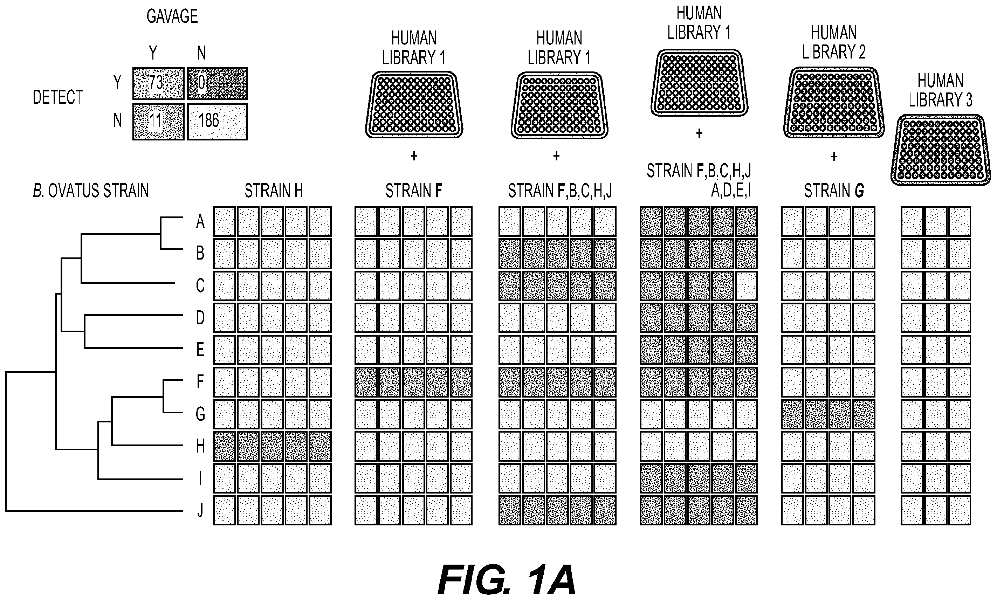

The inventors first confirmed the ability of *Strainer* to accurately detect the bacterial strains in gnotobiotic mice sequentially gavaged with defined culture collections of bacteria isolated from 3 different human fecal samples and a subset of 10 unique strains of the common human gut commensal bacterium *Bacteroides ovatus* **(****Figure 1A****).** The inventors quantified the overall performance in these simplified communities using precision and recall, which were 100% and 86.9% respectively, with no false positives in 280 different tests (specificity 100%).

### Example 4: Benchmarking the performance of metagenomics algorithms.

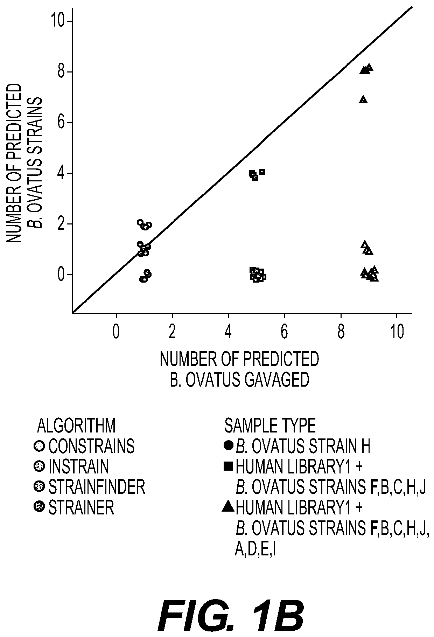

In the present disclosure, the dataset of strains isolated from matched and metagenomically sequenced FMT samples provides for the first time an *in vivo* experimental benchmark for rigorous comparison of SNP based inference approaches for tracking SNP strain proxies in metagenomics. The inventors tested the previously published *Strain Finder, ConStrains* and *inStrain* algorithms on the present gnotobiotic mice dataset. These SNP proxy algorithms, that were developed on synthetic and *in vitro* datasets, are inferior to the present disclosure because these proxy algorithms must first infer the strains from the metagenomes themselves. Any strain not inferred leads to false negatives across the dataset, and any strain incorrectly inferred propagates false positives in any sample where it is falsely detected. An illustration of the difference between the SNP proxy algorithms and the present disclosure can be seen by comparing the relative abilities of the different algorithms to estimate the correct number of *B. ovatus* strains in each mouse. Each of the SNP proxy algorithms struggled to do so; however, the ability of the *Strainer* algorithm of the present disclosure to detect the correct number of strains generated results that were in line with the actual number of *B. ovatus* strains gavaged into the mice **(****Figure 1B****,** R² = 0.99 for *Strainer*)*.*

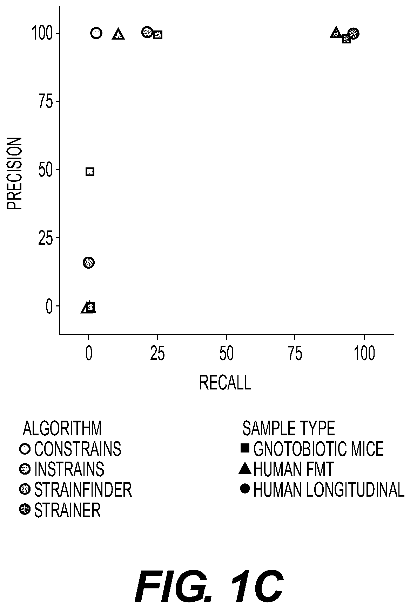

Confirmation of the difference in performance between the proxy algorithms and *Strainer* was obtained by examining whether the inferred strains match those gavaged to the germ-free mice. To make the comparison, the inventors provided the raw unassembled sequencing reads (~2.1M) for every strain (from its pure culture) as a distinct metagenomic "truth" sample and examined whether any of the algorithms could match the unassembled strain reads with the correct, corresponding metagenomics sample. None of the SNP proxy algorithms was able to do so **(****Figure 1C****).** But the *inStrain* algorithm was able to correctly identify the unassembled reads from different strain genomes as being distinct. Here, the sensitivity of *ConStrains* and *Strain Finder* algorithm was 0, while for *inStrains* it was 10.1. Meanwhile, the sensitivity of the present disclosure was 88.7. All algorithms had no false positives.

### Example 5: Strainer validation on complex human gut microbiotas.

To evaluate *Strainer* further, the inventors tested it in the context of several complex human gut microbiota communities with high species overlap but little to no strain overlap. This is a representative model for the use-case application for FMT where a potentially transmitted bacterial strain must be precisely detected across multiple individuals, while differentiating it from other related commensal strains from the same species. The inventors sequenced the fecal metagenome of 10 unrelated individuals as well as the genome of 261 bacterial strains isolated from the same fecal samples and then evaluated the ability of *Strainer* to detect these strains in the correct individual's metagenome, while not falsely detecting it in the other nine other samples.

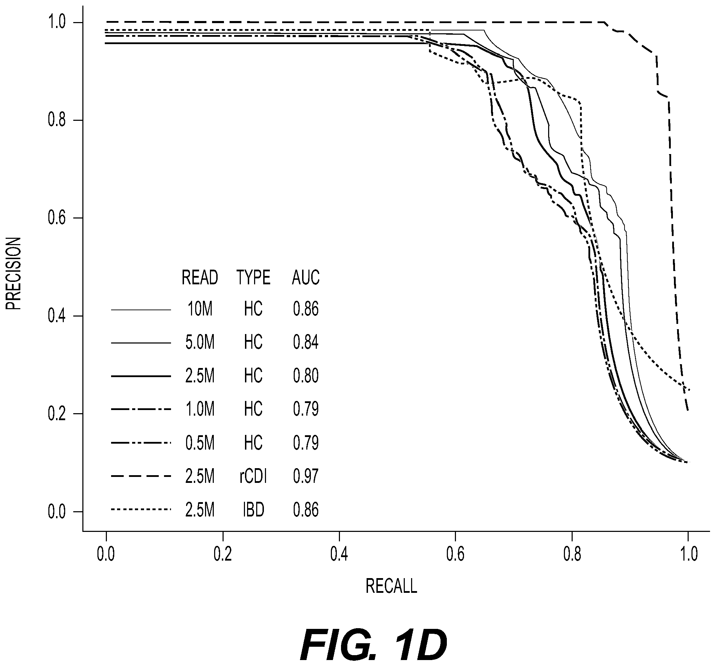

With 10M metagenomic reads per sample, the inventors reached a precision of 93.9% at a recall of 72.4% with an AUC of 0.86 **(****Figure 1D****).** The inventors attained slightly higher recall with deeper metagenomics; they found that even 500K metagenomic sequencing reads were sufficient to reach a precision of 95.8% with a recall of 57.6% **(****Figure 1D****).** The inventors generated similar testing datasets from five individuals with rCDI and four with IBD and found slightly higher AUC for rCDI as a result of the low diversity of the gut microbiome in rCDI **(****Figure 1D****).** While the inventors found high overall AUC, they also discovered that it was easier to detect some taxonomic orders than others using the present disclosure **(Table 2)**. The inventors discovered reduced performance for those species with smaller numbers of available reference genomes from which to infer informative k-mers, and for those species isolated by highly selective culture enrichments where culture is more sensitive than metagenomics. Together these results demonstrate that the present disclosure can accurately track sequenced bacterial strains in a metagenome, thus allowing quantification of discrete donor strain transmission in FMT.

**Table 2. Performance of Strainer sub-classified by different taxonomic order on the set of 261 strains and 10 different metagenomics samples presented in Figure 1D.**

| **Category** | **Precision** | **Recall** | **No. of Strains** |
|---|---|---|---|
| With 10 M reads | 93.9 | 72.4 | 261 |
| With 500K reads | 95.8 | 57.6 | 261 |
| | | | |

| **Order** | | | |
|---|---|---|---|
| Bacteroidales | 98.9 | 92.8 | 97 |
| Clostridiales | 88 | 54.3 | 81 |
| Bifidobacteriales | 92.3 | 85.7 | 28 |
| Lactobacillales | 83.3 | 35.7 | 27 |
| Enterobacterales | 83.3 | 58.8 | 17 |
| Coriobacteriales | 100 | 100 | 5 |

### Figure 1. Strainer algorithm accurately detects bacterial strains from complex gut communities and outperforms SNP-inference based metagenomics approaches.

**(A)** *Strainer* can accurately detect the correct *Bacteriodes ovatus* strain(s) in gnotobiotic mice, from other closely related strains. Each column represents an independent germ-free mouse gavaged with the specific B. ovatus strain(s) with or without a diverse human gut bacterial culture library of strains. Strains F and G were contained in human culture library 1 and 2 respectively. Human culture library 3 contained no *B. ovatus,* while the remaining *B. ovatus* isolates were isolated from other human fecal samples. Green box indicates the strain was introduced in the mice and detected in metagenomics (true positive), Grey indicates the strain was not detected and (true negative), Orange indicates the strain was detected but was not introduced (false positive) and Yellow indicates the strain was not detected but was gavaged in the mice (unknown as gavaging a strain does not always lead to stable colonization).

**(B)** Performance of SNP-inference based strain detection algorithms, *ConStrains, Strain Finder, inStrain* and our *Strainer* approach on detecting the number of *Bacteriodes ovatus* strain(s) in gnotobiotic mice.

**(C)** Precision-Recall curves to assess the performance of SNP-inference based strain tracking approaches and *Strainer* on real datasets ranging from sequential gavaging of a defined set of strains in gnotobiotic mice, FMT donor recipient pairs, and tracking the strain stability in a healthy individual over time.

**(D)** Performance assessment of *Strainer's* ability to match strains to the metagenome of the sample from which they were isolated. Solid lines denote the results at different sequencing depth after application of our algorithm on 261 strains isolated from healthy controls (HC). The color blue indicates the sequencing depth of 2.5M reads, while the dashed line indicates the result after application of *Strainer* on 56 strains isolated from patients with rCDI and the dotted curve is for 54 strains from patients with IBD. AUC of the Precision-Recall curves is in the legend box.

### Figure 4. The Strainer algorithm and comprehensiveness of the cultured bacterial strain library.

**(A)** Proportion of bacterial reads in the metagenomics sample that are explained by the genome sequences of the cultured strain library for that sample. Each point in the boxplot corresponds to a separate sample.
**(B)** Proportion of bacterial reads explained by the cultured strain library for a donor after gavaging (n = 3) germ-free mice with stool from (n = 3) corresponding human donors and performing metagenomics on the mouse fecal samples. Each point corresponds to a separate sample.
**(C)** Percentage similarity between 96 different isolates of species *Bacteriodes ovatus* and the reference strain AAXF00000000.2. Similarity is found by comparing sequence k-mers of length 31 between genomes.
**(D)** Proportion of bacterial reads in the metagenomics sample that are explained by the genome sequences of the cultured strain library for that sample. Each point in the boxplot corresponds to a separate sample.
**(E)** Overview of our algorithm *Strainer.*

The algorithm has 3 modules, where Module-1 involves finding the unique and likely informative sequence k-mers for each strain by removing those shared extensively with unrelated sequenced strains in NCBI, unrelated metagenomics samples, and those cultured and sequenced in this study. Next, the inventors decompose each sequencing read in the metagenomics sample of interest into its k-mers, and find reads that have k-mers belonging to multiple strains, or have <95% of informative k-mers for a single strain. The inventors further remove these non-informative k-mers from the previous set. In Module-2, the inventors assign sequencing reads from the metagenomics sample of interest, with a majority of informative k-mers (>95%) to each strain. Next, the inventors map these reads to the genome of the corresponding strain, and consider the non-overlapping ones only. This step normalizes for sequencing depth across samples and checks for evenness of read distribution across the bacterial genome. Finally, in Module-3 the inventors compare the read enrichment in a sample to unrelated samples or negative controls and present summary statistics for presence or absence of a strain in a sample.

### Example 6: FMT strain dynamics in rCDI patients.

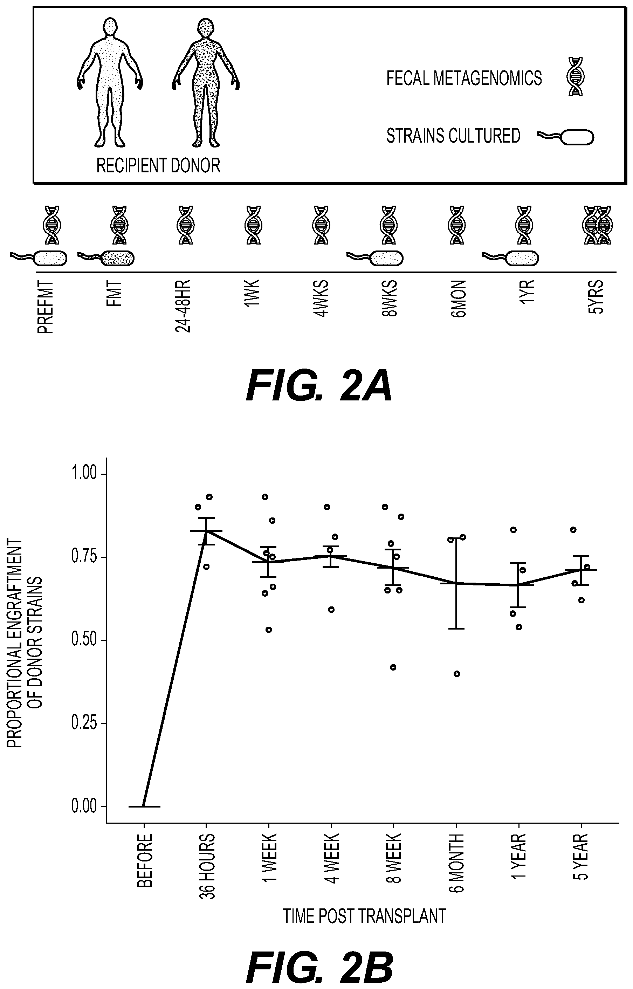

**Engraftment in FMT recipients:** In the clinical cohort, seven FMT donors each provided their sample to a single recipient (which was sampled at multiple timepoints post-FMT), while one donor provided the sample to seven different patients **(Table 1,** **Figure 2A****).**

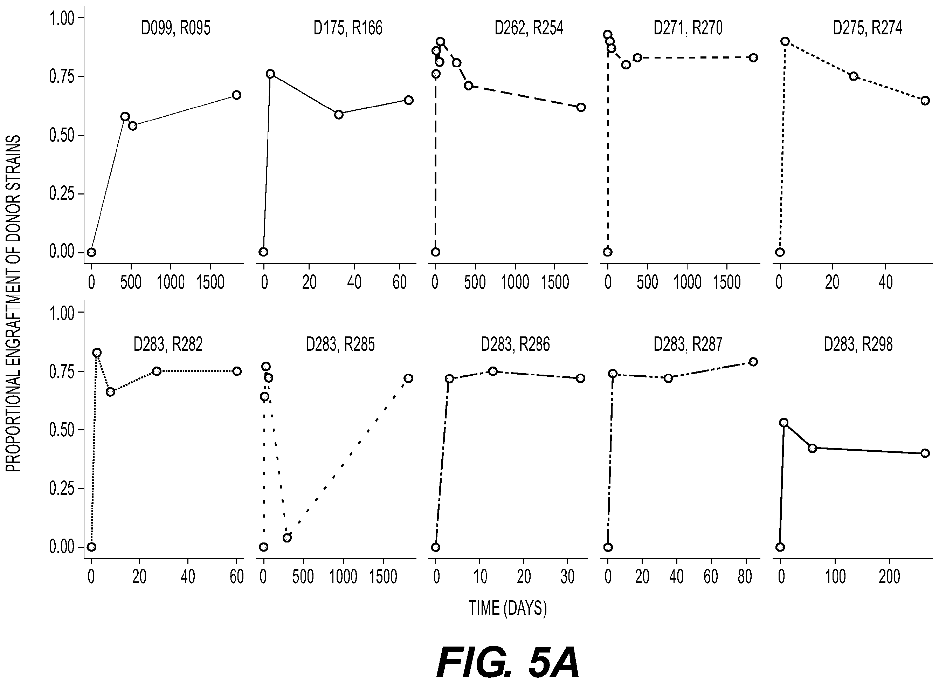

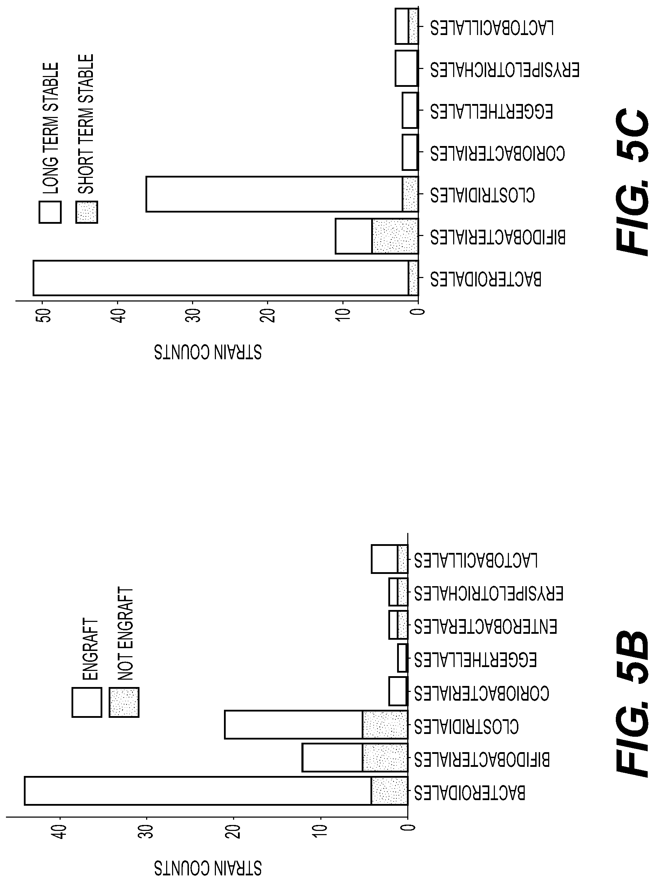

Previous FMT approaches demonstrated sharing of microbiota between the donor and the recipient post-FMT, but none has demonstrated precise quantification of engraftment. The inventors used *Strainer* to measure the engraftment of donor strains in the recipients and defined the Proportional Engraftment of Donor strains (PED) metric as the number of donor strains detected in a recipient post-FMT divided by total number of strains isolated from the donor. The inventors tracked 10 non-relapsing recipients for up to five years after FMT and found consistently high engraftment of donor strains at all time points **(****Figure 2B****,** individual trajectories for donor-recipient pairs in **Figure 5A****).** In these individuals, the inventors found an average engraftment of 83% (sd = 9%) at 36 hours, which stabilizes at 71% (sd = 16%) at 8 weeks (a common clinical end point for measuring efficacy) and remains consistently high at 71% (sd = 9%) even 5 years later. This demonstrates that gut microbiota manipulation by FMT can lead to a near permanent engraftment of a new stable set of bacterial strains in patients with rCDI. The inventors discovered that strains belonging to order Bifidobacteriales engrafted less at 8 weeks (67% of strains), while strains in order Bacteriodales engrafted higher (92% of strains, **Figure 5B****),** and the inventors observed very little engraftment from order Lactobacillales.

The inventors found that 50 out of 51 strains belonging to order Bacteriodales, which engrafted at 8-weeks, remained stably engrafted at a longer time-scale of 6-months or more **(****Figure 5C****).** However, fewer strains belonging to order Bifidobacteriales, which engrafted at 8-weeks, remained stably engrafted at 6-months or longer timescale (only 5 out of 11, p-val < 10⁻⁵ fisher-exact test).

### Example 7: Validation of bacterial strain engraftment through culturing.

The isolation and sequencing of the transmitted strains from both the donor and the recipient represents a gold standard validation and verification of the *commensal* Koch's postulates. To date, there is no large study demonstrating transmission of donor bacterial strains from multiple species and across different FMT interventions by culture. The inventors cultured strains from 6 recipients both pre- and post-FMT **(****Figure 2A****)** and compared the strain composition to that from the donor, to experimentally validate bacterial strain transmission. The inventors did not isolate a single donor strain in any recipient prior to transplant, yet they isolated 48 donor strains in recipients post-FMT, encompassing 16 different species **(Table 3).**

**Table 3. Gold standard set of bacterial strains cultured and isolated independently both from the donor and recipient post-FMT demonstrating transmission.**

| Species | No. of strains cultured for this species | Cultured in both donor and recipient post-FMT at 8 weeks | Cultured in both donor and recipient post-FMT at 1 year |
|---|---|---|---|
| *Bacteroides ovatus* | 7 | 6 | 1 |
| *Bacteroides vulgatus* | 7 | 6 | 1 |
| *Bifidobacterium longum* | 4 | 4 | |
| *Alistipes finegoldii* | 3 | 3 | |
| *Bacteroides uniformis* | 3 | 3 | |
| *Bifidobacterium bifidum* | 3 | 2 | 1 |
| *Parabacteroides distasonis* | 3 | 3 | |
| *Parabacteroides merdae* | 3 | 3 | |
| *Bacteroides caccae* | 2 | 2 | |
| *Bacteroides thetaiotaomicron* | 2 | 2 | |
| *Bifidobacterium adolescentis* | 2 | 2 | |
| *Bifidobacterium pseudocatenulatum* | 2 | 2 | |
| *Collinsella aerofaciens* | 2 | 2 | |
| *Odoribacter splanchnicus* | 2 | 1 | 1 |
| *Bacteroides cellulosilyticus* | 1 | 1 | |
| *Bacteroides fragilis* | 1 | 1 | |
| *Butyricimonas faecalis* | 1 | 1 | |

The vast majority of these (46/48) strains were also detected independently in metagenomics samples from the same timepoint when they were cultured, and the other 2 were detected at an earlier timepoint, highlighting the *Strainer* algorithm's capability to track and study engraftment of strains post-FMT.

The inventors quantified tracking performance on these gold standard strains (which were isolated either in one person across multiple timepoints, or between the donor and the recipient using different algorithms) and found that the present disclosures method for FMT tracking had overall sensitivity of 92.9 (with 1 false positive) while *inStrain* had 25.3, *Strain Finder* had 0 and *ConStrains* had 1.4 **(****Figure 1C****).** For tracking in longitudinally cultured samples, the present disclosure had overall sensitivity of 96.6 (with no false positive) while *inStrain* had 21.8, *Strain Finder* had 0 and *ConStrains* had 3.4. This comparison on human gold standard experimentally verified strain transmission datasets demonstrates for the first time that the present disclosure is capable of tracking longitudinally cultured samples and FMT.

### Example 8: FMT results in loss of original resident strains.

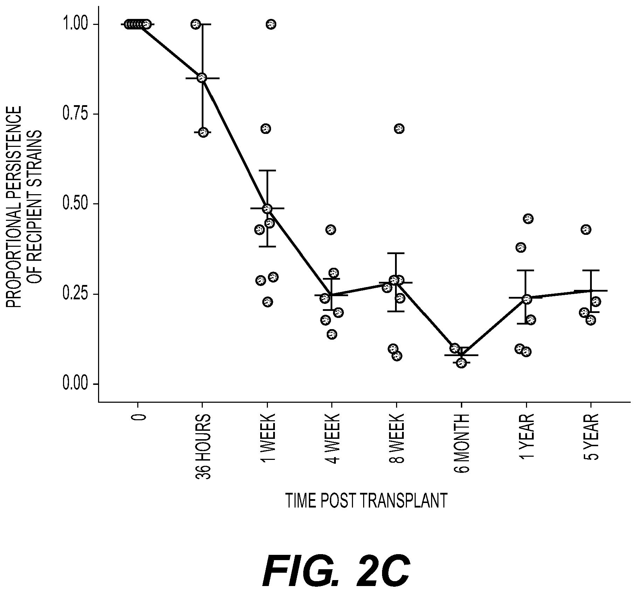

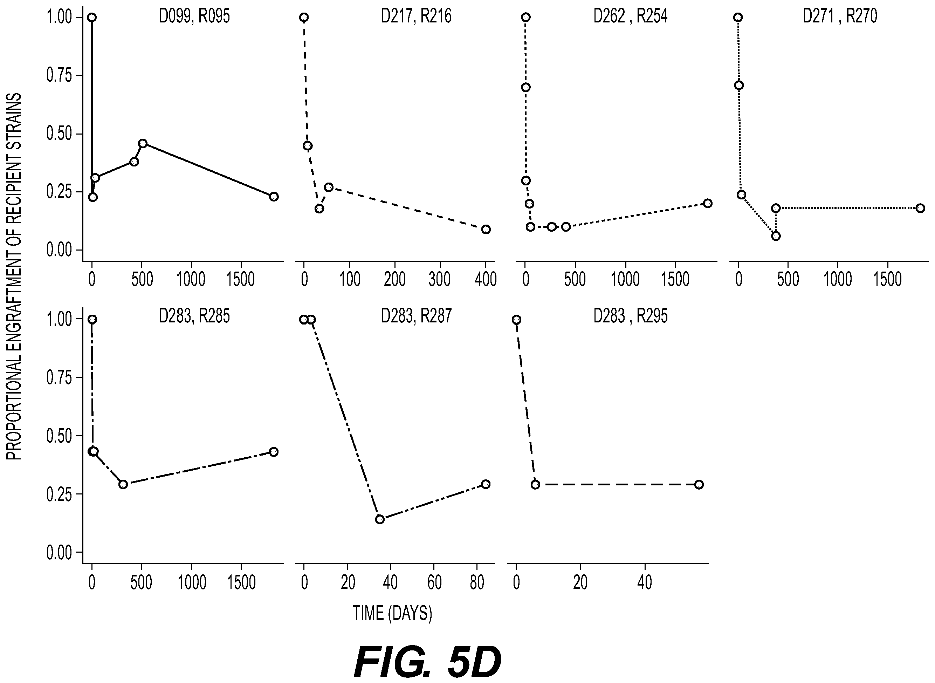

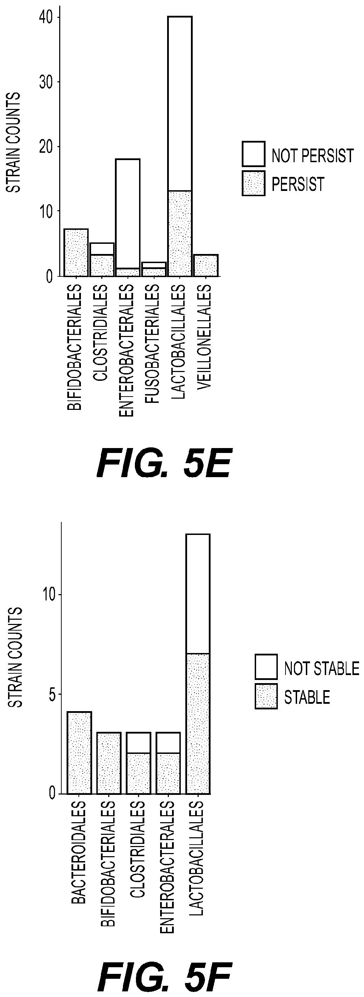

Studies have shown that resident microbiota strains create ecological niches, which in turn can influence the engraftment of donor microbes post-FMT. Thus, it is important to identify the bacterial strains present pre-FMT and resolve their persistence dynamics after transplantation. Here, the inventors isolated and sequenced the pre-FMT resident strains in 7 recipients and tracked them for up to 5 years in each recipient's metagenome. Similar to the PED metric, the inventors defined Proportional Persistence of Recipient Strains (PPR) as the ratio between the strains of the recipient observed post-FMT to total recipient strains cultured pre-FMT. Unlike the rapid high engraftment of donor strains, the inventors found a more graduated decline in the PPR **(****Figure 2C****,** individual trajectories for donor-recipient pairs in **Figure 5D**) with the overall persistence decreasing to 49% (sd = 28%) at 1 week and 21% (sd = 10%) at 8 weeks (P val < 0.02 from Wilcox test). The inventors found that the recipient strains belonging to order Bifidobacteriales consistently persisted (7 of 7) in the recipients for 8 weeks post-FMT **(****Figure 5E****).** Recipient strains from order Lactobacillales and Enterobacterales, however, were largely eliminated by the FMT. As observed in previous studies, the inventors observe an instability of the recipient's gut microbiota; however, the inventors discovered that a subset of the original strains remain durable over time.

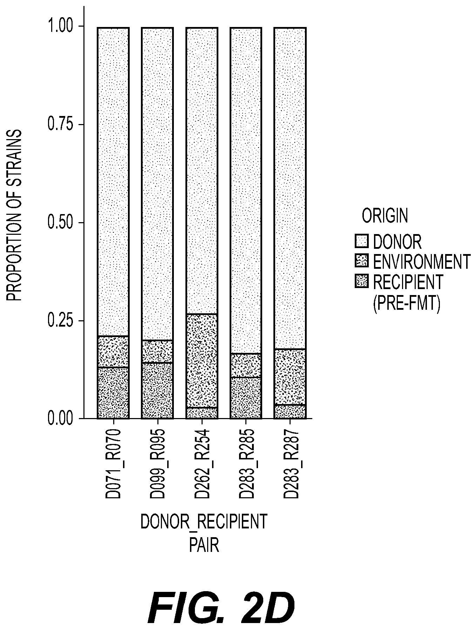

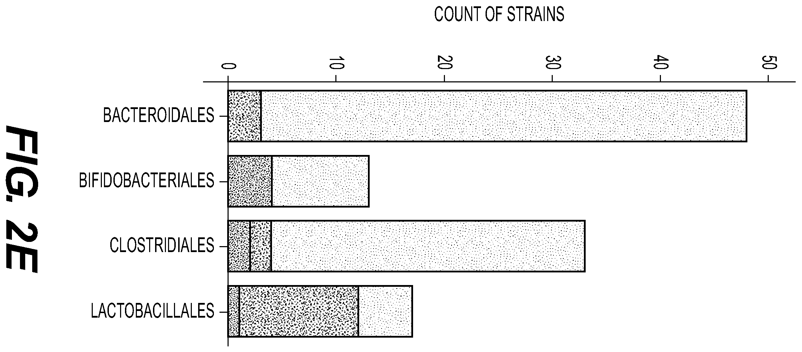

### Figure 2. FMT strain dynamics in recipients after a single dose of FMT for up to 5 years.

**(A)** Overview of FMT study design indicating the dates of metagenomic sequencing and bacterial strain culturing. The genome sequences of the cultured bacterial strains are used to track each strain across metagenomic samples using *Strainer.*
**(B)** Strains from the donor remain stably engrafted in successful post-FMT patients for at least 5 years after transplant.
**(C)** Strains isolated from a recipient prior to FMT are rapidly lost with a small proportion persisting at longer timescales.
**(D)** Proportion of donor, recipient, and environment strains detected in patients post-FMT. Environmental strains are non-donor and non- recipient (prior to FMT) in origin, which are both cultured and metagenomically detected post-FMT.
**(E)** Count of strains detected in patients post-FMT subclassified by major phylogenetic taxa (at order level) and colored based on their origin.

### Example 9: Engraftment of non-donor strains after FMT.

The inventors investigated whether donor and pre-FMT recipient strains lead to complete niche occupancy of the host, or whether there is further engraftment of gut microbes from other individuals and environmental sources. The inventors isolated and tracked strains from 5 subjects post-FMT and found 24 strains that were non-donor and non-recipient in origin that were metagenomically detected and cultured in recipients post-FMT. On average in a patient post-FMT, 8.9% strains persisted from the recipient pre-FMT, 79.6% strains engrafted from the donor, and 11.5% strains were non-donor or non-recipient in origin **(****Figure 2D****).** Although their origin and mode of transfer remains unknown, these environmental strains belong to phylogenetic taxa detected in both healthy donors and recipients prior to FMT **(****Figure 2E****)** with similar colonization patterns **(****Figure 5F****).** These results suggest that approximately 11.5% of the recipient niche space is stably colonized by other sources and that LBPs with more limited niche occupancy will require a larger acquisition of environmental microbes for the host to become fully colonized.

### FIGURE 5. FMT strain dynamics (donor, pre-FMT recipient and novel environmental strains) in recipients post-FMT.

**(A)** Trajectory of proportional strain engraftment of donor strains in each recipient at all available timepoints (in days). The donor recipient pair ids are at the top of each plot.
**(B)** Number of strains that transmit and engraft for at least 8-weeks in patients post-FMT (single FMT donor to recipient setting) grouped by taxonomic order.
**(C)** The number of strains colonized at 8 weeks (short term) that engraft for at least 6-months or more (long-term) in patients post-FMT (both single FMT donor to single and multiple recipients setting) grouped by taxonomic order.
**(D)** Trajectory of proportional persistence of recipient's strains post-FMT at all available timepoints (in days). The donor recipient pair ids are at the top of each plot.
**(E)** The number of the recipient's original strains that persist for at least 8-weeks post-FMT, grouped by taxonomic order.
**(F)** The number of environment strains (i.e. non-donor and non-recipient in origin) that engraft in patients stably over multiple timepoints (>1 week) post-FMT, grouped by taxonomic order.

### Example 10: Donor engraftment independently explains rCDI FMT clinical outcomes.

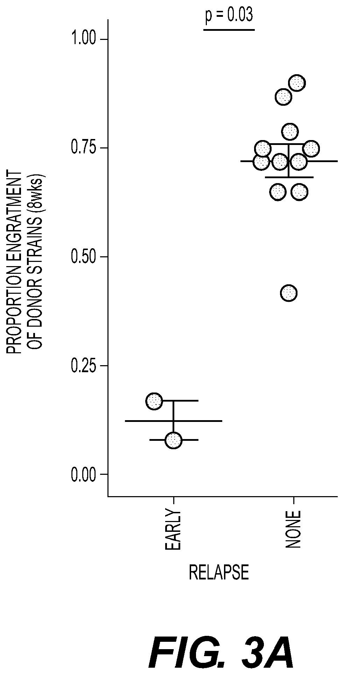

Eight weeks is the typical timepoint for evaluating the efficacy of FMT interventions, which can be accomplished by comparing the number of patients that achieved the clinical endpoint with those that failed to do so. PED provides a potential quantitative surrogate marker to understand FMT clinical success or relapse. In the two patients in this cohort who experienced an early relapse within 8-weeks of FMT, the inventors found significantly reduced PED **(****Figure 3A****,** p-val=0.03 from two-sided Wilcox test) compared to those that successfully achieve the clinical endpoint of no rCDI recurrence at 8-weeks post-FMT. This result reveals that precise engraftment of donor strains in recipients can independently explain the early clinical outcome of an FMT intervention, as subjects could be perfectly classified into relapse or non-relapse with a PED threshold of 17%.

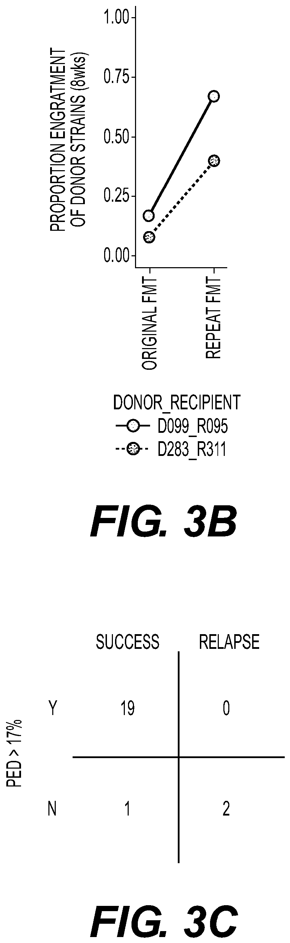

Individuals that undergo repeat FMT often respond to treatment the second time. Therefore, the inventors evaluated if the present PED metric can elucidate the outcome of repeat-FMT in such patients. The 2 recipients (R095 and R311) that had an early failure, received a repeat dose of FMT and reported clinical success (i.e., no relapse with rCDI recurrence) at future timepoints (including at 5 year for R095). The inventors found that PED was significantly higher after the repeat dosage **(****Figure 3B****).**

Since PED was able to explain both relapse and outcome of repeat-FMT in patients, the inventors evaluated the overall predictive power of the present disclosure on all available FMT samples where clinical evaluation was independently noted **(****Figure 3C****).** The inventors found that wherever there was clinical success (i.e., no relapse), they also found engraftment to be above the threshold of 17% (n = 19 true positives) with 1 false negative. Similarly, clinical relapse was always independently associated with low engraftment (n = 2 true negatives) with no false negatives. Together, these results show that engraftment of donor strains at any time point can provide an accurate and robust metric (precision = 100%, sensitivity = 95%) for independently explaining the clinical outcome of FMT, both for initial and after a repeat FMT.

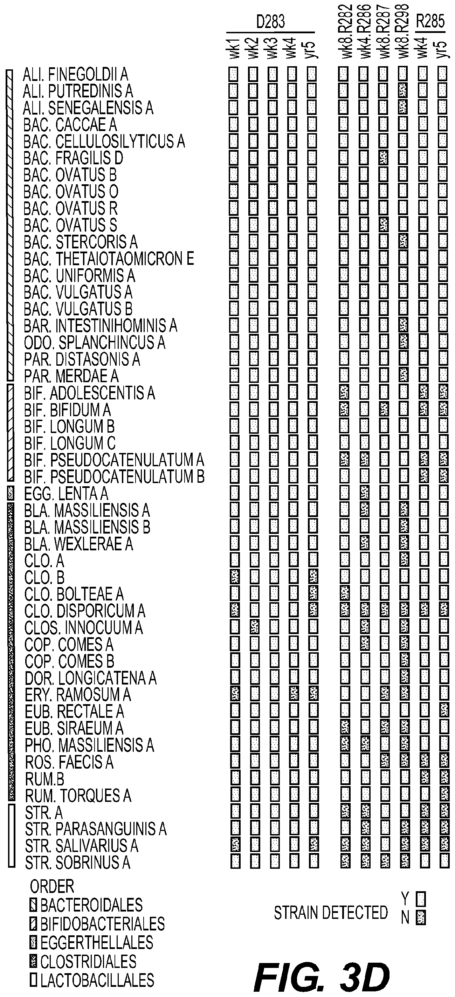

### Figure 3. Donor engraftment independently explains rCDI FMT clinical outcomes and identifies bacterial strains for LBP.

**(A)** Proportional Engraftment of donor's (PED) strains at 8-weeks can predict early relapse of FMT in patients with rCDI.
**(B)** PED metric can elucidate the successful outcome of repeat-FMT in patients that relapsed with rCDI after the initial-FMT.
**(C)** Predictive power of our approach on all available FMT samples where clinical evaluation was independently noted. Whenever we report clinical success we find engraftment to be above the threshold of 17% (n = 19 true positives) with 1 false negative. Clinical relapse was always independently associated with low engraftment (n = 2 true negatives) with no false negatives
**(D)** Bacterial strain engraftment and identification of highly transmissible strains that stably engraft in multiple recipients. The first 4 columns are weekly metagenomic samples from the donor, while the 5^{th} column is the donor sample from 5 years later. The next 6 columns are from the FMT recipients that did not have an early relapse. The last column is from one of the recipient 5 years later. *Strainer* was used to find the presence (green) or absence (yellow) of each bacterial strain from the corresponding metagenomics sample.

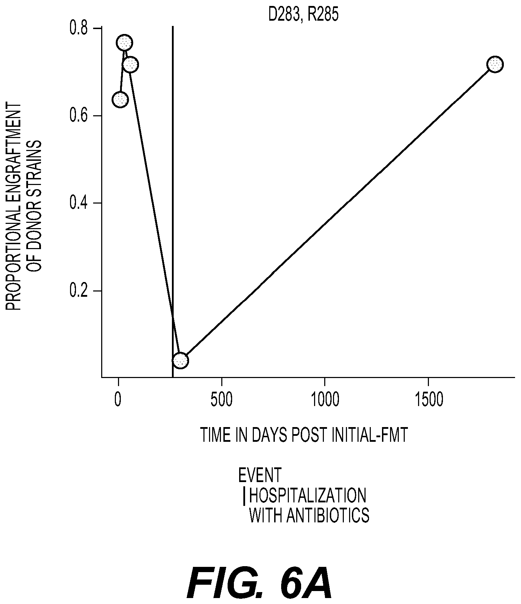

The inventors did find one case of very low engraftment in an otherwise successful FMT with no relapse occurred in patient R285 **(****Figure 6A****).** This patient reported high engraftment of 77% at day 30, 72% at day 58, reduction to 4% on day 300, and increased again 5 years later to 72%. The patient was symptom free at both 2 months and 5 years, in sync with expectations due to higher engraftment at those timepoints, which is why the low engraftment was initially surprising. However, this patient was hospitalized with severe diarrhea and antibiotics on day 258 post-FMT, which perhaps explains the low PED measured in their metagenome on day 300, although this would suggest their microbiome had not recovered over a relatively substantial period of 42 days. Importantly, this individual was not given a repeat FMT, suggesting the lower engraftment at day 300 post-FMT resulted in the large majority of engrafted strains being reduced below the detection limit of our algorithm but not being eliminated from the gut.

### Example 11: Identification of bacterial strains for LBP.

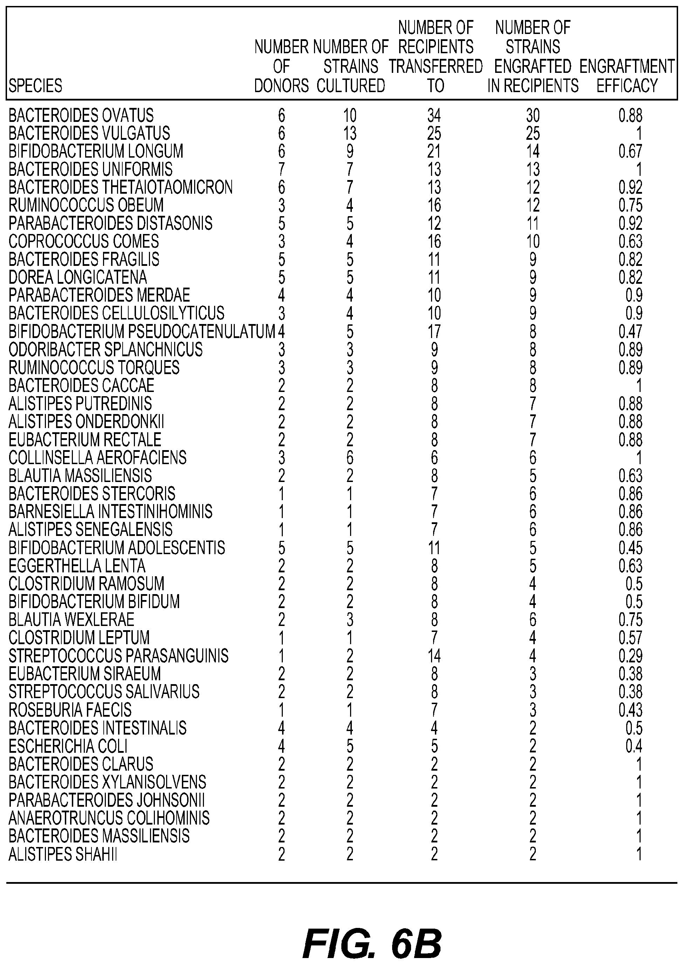

The inventors have developed a consortium of culturable, discrete strains for use in LBPs as a safer, scalable alternative to FMT. The inventors have demonstrated for the first time a consortium of a transferable, culturable engrafting fraction of human-tested donor fecal microbiotas, where strains that do not transfer are eliminated, and multi-drug resistant organisms (MDROs) are removed. Donor D283 was used for multiple (n = 5 non-relapsing) recipients, thus providing more power to detect engraftment consistency of single strains **(****Figure 3D****).** Focusing on the highly transmissible strains that stably engraft in at least 4 out of 5 non-relapsing recipients, the inventors found that those belonging to order Bacteriodales always engrafted (100%, 19/19, even up to 5 years), showing that these strains and others that stably engraft for longer duration in successfully treated patients can be included in LBPs. The inventors also provide a comprehensive list of species from all donors and the frequency at which strains from each species engraft in recipients **(****Figure 6B****).** These engrafting strains and species provide validated components for use in additional LBP compositions.

### Figure 6.

**(A)** Engraftment of donor D283 strains in recipient R285, which did not relapse but rather had a temporary loss in detectability of the donor strains during antibiotic treatment for severe diarrhea.
**(B)** Identification of a set of bacterial species for LBP, based on their culturing and engrafting efficacy across recipients. "Number of donors" correspond to the donors where strains from this species have been cultured or detected metagenomically. "Number of strains cultured" represents the unique strains cultured and metagenomically detected for this species. "Number of recipients transferred to" corresponds to number of FMT recipients (counted separately for each strain cultured from this species) which received a strain from this species. "Number of strains engrafted in recipients" represents the strains that engrafted for at least 8-weeks (a common clinical endpoint) in a recipient. "Engraftment efficacy" is calculated as the ratio of "Strain engraftment/Column 5" and "Recipients transferred to/Column 4".

### Example 12: Generating compositions suitable for human trials.

To be suitable for human trials, the strains in the bacterial consortium must be cultivatable in growth media that is free of animal products. The inventors discovered that all 16 bacterial strains can be cultured in a specific animal free media LYH_VIB (Table 7). All strains reach sufficient optical density (OD₆₀₀) and potency (CFU/mL) cultured in LYH_VIB to be manufactured for human trials (Table 5). For safety considerations, the inventors focused on bacterial consortium strains that would be susceptible to multiple antibiotics. The inventors tested susceptibility to a range of antibiotics for all strains included in MTC01 and the minimal inhibitory concentration (MIC) was determined according to guidelines of the Clinical and Laboratory Standards Institute (CLSI). All strains were susceptible to multiple antibiotics (Table 6). A further consideration for the manufacture of these strains is the need to identify potential contaminant bacteria within the drug, most notably facultative anaerobic pathogens. USP<61> is an established assay for testing if a product is contaminated or does not have a high number of aerobic bacteria, yeast, and fungi in it. To apply this test in the context of a drug composed of bacteria, it is important that the bacteria are not aerobic or facultative aerobic organisms and that the drug strains do not inhibit the growth of other aerobic of facultative organisms used in the USP<61> assay. The inventors confirmed that all 16 strains were strict anaerobes with no bacterial growth documented for any of the strains under aerobic conditions as confirmed by total aerobic microbial count (TAMC). The inventors also confirmed that none of the 16 strains inhibited the growth of the USP<61> control organisms, *S. aureus* (ATCC6538); *P. auruginosa* (ATCC9027), *B. subtilis* (ATCC6633), *C. albicans* (ATCC10231) and *A. brasiliensis* (ATCC16404), as >50% recovery was demonstrated for these control organisms when incubated aerobically with each of the 16 therapeutic strains.

**Table 4. Composition of the animal free medium LYH_VIB.**

| **Component** | | | **Amount [g/L]** |
|---|---|---|---|
| Vegitone infusion broth | | | 37 |
| Yeast extract | | | 5 |
| Monosaccharide mix | | | 4 |
| | - D-xylose | | 1 |
| | - D-fructose | | 1 |
| | - D-glucose | | 1 |
| | - D-galactose | | 1 |
| | - N-acetylglucosamine | | 0.5 |
| | - L-arabinose | | 0.5 |
| Disaccharide mix | | | 3 |
| | - D-Cellobiose | | 1 |
| | - D-Maltose | | 1 |
| | - Sucrose | | 1 |
| L-cysteine hydrochloride | | | 0.5 |
| L-Malic acid | | | 1 |
| Sodium sulfate | | | 2 |
| MOPS | | | 20.9 |

| | | | **Volume [mL/L]** |
|---|---|---|---|
| Vitamin-K solution (1 mg/mL) | | | 1 |
| Tween 80 | | | 0.5 |
| H₂O | | | Adjust to 1 L |
| | | | pH 7.2 (NaOH) |

**Table 5. Optical density (OD₆₀₀) and potency (CFU/mL) of bacterial strains included in MTC01, cultured in LYH_VIB animal free medium. OD₆₀₀ measurements are undiluted.**

| **Strain** | **OD₆₀₀** | **CFU/mL** |
|---|---|---|
| MTC01.01_*B*. *uniformis* | 1.3 | 5.05E+0 9 |
| MTC01.02_*B*. *ovatus* | 1.4 | 9.6E+08 |
| MTC01.03*_B. longum* | 1 | 2E+08 |
| MTC01.04_*B*. *thetaiotaomicron* | 1.5 | 3.65E+0 9 |
| MTC01.05_*B. vulgatus* | 1.15 | 3.67E09 |
| MTC01.06*_C. aerofaciens* | 1.1 | 1.03E+0 9 |
| MTC01.07*_P. distasonis* | 1.3 | 6.5E+09 |
| MTC01.08_*B*. *adolescentis* | 1.4 | 2E+09 |
| MTC01.09*_P. merdae* | 0.7 | 1.3E+09 |
| MTC01.10*_C. comes* | 1.5 | 5.75E+07 |
| MTC01.11*_E. rectale* | 1.2 | 1.75E+09 |
| MTC01.12_*B*. *caccae* | 1 | 8.6E+08 |
| MTC01.13_*D. longicatena* | 1.4 | 3.75E+08 |
| MTC01.14_*O. splanchnicus* | 0.9 | 3.3E+09 |
| MTC01.15_*B*. *cellulosilyticus* | 1.58 | 4.5E+09 |
| MTC01.16_*B*. *pseudocatenulatum* | 1.2 | 1.07E+09 |

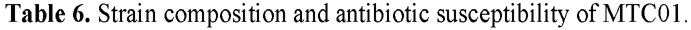

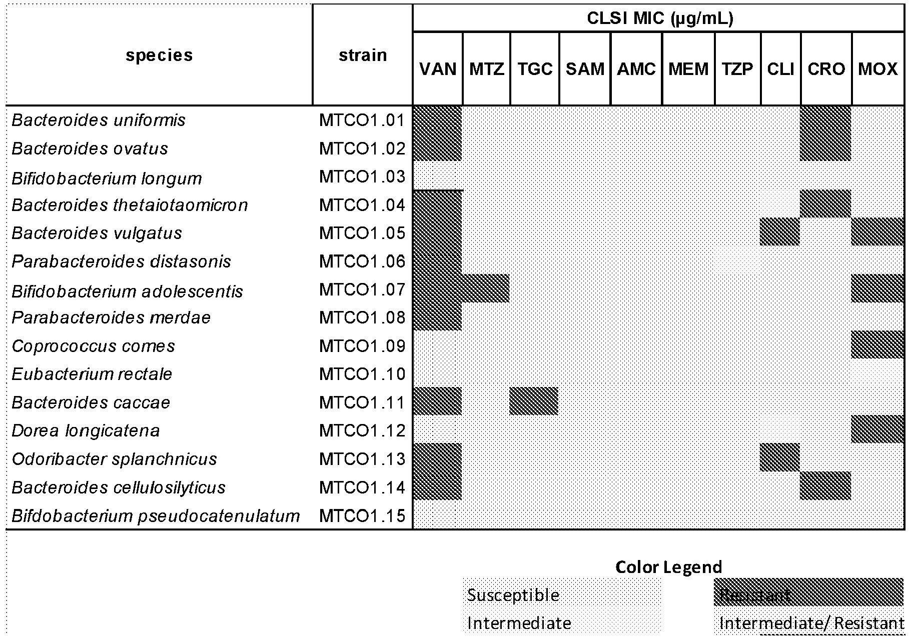

Each strain is susceptible to multiple antibiotics, and all strains are susceptible to three antibiotics (SAM, AMC, MEM). Minimum inhibitory concentrations (MIC) were determined by a CRO [Micromyx, LLC] according to CLSI standards and in-house by etest, keeping the highest value between the two methods: vancomycin (VAN), metronidazole (MTZ), tigecycline (TGC), ampicillin/sulbactam (SAM), amoxicillin/clavulanic acid (AMC), meropenem (MEM), piperacillin/tazobactam (TZP), clindamycin (CLI), ceftriaxone (CRO), moxifloxacin (MOX). All strains failed to grow aerobically in a USP<61> assay but did not inhibit the growth of positive control organisms validating USP<61> as a release assay for the master cell banks and drug product. Finally, multiple test fermentations were used to determine that the volumes for each manufacturing run are well within the 8L capacity of our current manufacturing set-up.

**Table 7. Bacterial strains, their accession numbers, and the percentage of k-mers remaining after removing previously seen k-mers.**

| **Strain name** | **Accession number** | **% of k-mers left after initial scrubbing** |
|---|---|---|
| Alistipes onderdonkii A | SAMN15532401 | 4.56 |
| Alistipes onderdonkii B | SAMN15532574 | 2.5 |
| Alistipes onderdonkii C | SAMN15532875 | 3.14 |
| Alistipes onderdonkii D | SAMN15533267 | 5.82 |
| Alistipes onderdonkii E | SAMN15533356 | 2.34 |
| Alistipes onderdonkii F | SAMN15533361 | 2.18 |
| Alistipes putredinis A | SAMN15532518 | 5.17 |
| Alistipes putredinis B | SAMN15532760 | 1.74 |
| Alistipes senegalensis A | SAMN15533282 | 2.97 |
| Alistipes senegalensis B | SAMN15532438 | 22.57 |
| Alistipes shahii A | SAMN15532555 | 3.19 |
| Alistipes shahii B | SAMN15532876 | 3.28 |
| Alistipes shahii C | SAMN15533117 | 3.38 |
| Alistipes shahii D | SAMN15533325 | 3.34 |
| Alistipes shahii E | SAMN15634165 | 2.99 |
| Anaerotruncus colihominis A | SAMN15532646 | 4.96 |
| Anaerotruncus colihominis B | SAMN15532688 | 5.68 |
| Anaerotruncus colihominis C | SAMN15532694 | 6.96 |
| Anaerotruncus colihominis D | SAMN15532706 | 4.96 |
| Anaerotruncus colihominis E | SAMN15533212 | 29.64 |
| Bacteroides caccae A | SAMN15532375 | 1.95 |
| Bacteroides caccae B | SAMN15532523 | 1.71 |
| Bacteroides caccae C | SAMN15532625 | 0.96 |
| Bacteroides caccae D | SAMN15532627 | 2.74 |
| Bacteroides caccae E | SAMN15532734 | 2.76 |
| Bacteroides caccae F | SAMN15532371 | 4.19 |
| Bacteroides caccae G | SAMN15532983 | 2.47 |
| Bacteroides caccae H | SAMN15533254 | 2.9 |
| Bacteroides caccae I | SAMN15533324 | 2.63 |
| Bacteroides cellulosilyticus A | SAMN15532683 | 5.47 |
| Bacteroides cellulosilyticus B | SAMN15532520 | 5.82 |
| Bacteroides cellulosilyticus C | SAMN15532565 | 5.3 |
| Bacteroides cellulosilyticus D | SAMN15532751 | 5.41 |
| Bacteroides cellulosilyticus E | SAMN15532769 | 1.41 |
| Bacteroides cellulosilyticus F | SAMN15532994 | 5.53 |
| Bacteroides cellulosilyticus G | SAMN15533005 | 8.83 |
| Bacteroides cellulosilyticus H | SAMN15533057 | 2.27 |
| Bacteroides cellulosilyticus I | SAMN15533083 | 4.22 |
| Bacteroides cellulosilyticus J | SAMN15533185 | 6.84 |
| Bacteroides cellulosilyticus K | SAMN15533306 | 3.58 |
| Bacteroides cellulosilyticus L | SAMN15533339 | 5.69 |
| Bacteroides cellulosilyticus M | SAMN15533341 | 6.42 |
| Bacteroides clarus A | SAMN15532473 | 11.92 |
| Bacteroides clarus B | SAMN15532689 | 13.1 |
| Bacteroides clarus C | SAMN15532714 | 15.42 |
| Bacteroides clarus D | SAMN15532989 | 16.78 |
| Bacteroides fragilis A | SAMN15532415 | 6.3 |
| Bacteroides fragilis B | SAMN15532905 | 11.52 |
| Bacteroides fragilis C | SAMN15532774 | 1.64 |
| Bacteroides fragilis D | SAMN15532440 | 2.34 |
| Bacteroides fragilis E | SAMN15532424 | 11.46 |
| Bacteroides fragilis F | SAMN15532632 | 5.8 |
| Bacteroides fragilis G | SAMN15532727 | 3.27 |
| Bacteroides fragilis H | SAMN15532816 | 5.13 |
| Bacteroides fragilis I | SAMN15532846 | 2.6 |
| Bacteroides fragilis J | SAMN15532926 | 3.17 |
| Bacteroides fragilis K | SAMN15532927 | 2.5 |
| Bacteroides fragilis L | SAMN15533184 | 2.53 |
| Bacteroides fragilis M | SAMN15533336 | 1.85 |
| Bacteroides fragilis N | SAMN15532377 | 2.82 |
| Bacteroides intestinalis A | SAMN15532975 | 19.9 |
| Bacteroides intestinalis B | SAMN15532980 | 10.24 |
| Bacteroides intestinalis C | SAMN15533040 | 10.37 |
| Bacteroides intestinalis D | SAMN15533140 | 7.69 |
| Bacteroides intestinalis E | SAMN15533217 | 22.83 |
| Bacteroides massiliensis A | SAMN15532452 | 2.68 |
| Bacteroides massiliensis B | SAMN15532475 | 6.29 |
| Bacteroides massiliensis C | SAMN15532515 | 2.46 |
| Bacteroides massiliensis D | SAMN15532567 | 2.04 |
| Bacteroides massiliensis E | SAMN15532693 | 7.48 |
| Bacteroides massiliensis F | SAMN15532804 | 1.5 |
| Bacteroides massiliensis G | SAMN15532958 | 2.41 |
| Bacteroides massiliensis H | SAMN15533069 | 5.7 |
| Bacteroides massiliensis I | SAMN15533205 | 4.54 |
| Bacteroides ovatus a | SAMN15532696 | 2.47 |
| Bacteroides ovatus A | SAMN15532859 | 2.61 |
| Bacteroides ovatus b | SAMN15532785 | 3.19 |
| Bacteroides ovatus B | SAMN15532699 | 2.11 |
| Bacteroides ovatus C | SAMN15654963 | 5.64 |
| Bacteroides ovatus c | SAMN15532799 | 2.43 |
| Bacteroides ovatus D | SAMN15533334 | 3.2 |
| Bacteroides ovatus d | SAMN15532906 | 1.9 |
| Bacteroides ovatus E | SAMN15532898 | 2.65 |
| Bacteroides ovatus e | SAMN15532941 | 3.02 |
| Bacteroides ovatus F | SAMN15533340 | 2.67 |
| Bacteroides ovatus f | SAMN15532999 | 3.37 |
| Bacteroides ovatus G | SAMN15533153 | 2.56 |
| Bacteroides ovatus g | SAMN15533080 | 1.79 |
| Bacteroides ovatus H | GCA_002959635.1 | 2.46 |
| Bacteroides ovatus h | SAMN15533082 | 2.33 |
| Bacteroides ovatus i | SAMN15533337 | 1.88 |
| Bacteroides ovatus I | SAMN15533245 | 3.71 |
| Bacteroides ovatus J | SAMN15654964 | 3.49 |
| Bacteroides ovatus K | SAMN15532753 | 3.11 |
| Bacteroides ovatus L | SAMN15532583 | 2.98 |
| Bacteroides ovatus M | SAMN15532829 | 2.33 |
| Bacteroides ovatus N | SAMN15533355 | 2.13 |
| Bacteroides ovatus O | SAMN15533044 | 1.11 |
| Bacteroides ovatus P | SAMN15533118 | 2.81 |
| Bacteroides ovatus Q | SAMN15532634 | 4.53 |
| Bacteroides ovatus R | SAMN15533222 | 2.71 |
| Bacteroides ovatus S | SAMN15532609 | 4.2 |
| Bacteroides ovatus T | SAMN15532418 | 4.06 |
| Bacteroides ovatus U | SAMN15532458 | 4.26 |
| Bacteroides ovatus V | SAMN15532462 | 2.89 |
| Bacteroides ovatus W | SAMN15532494 | 1.85 |
| Bacteroides ovatus X | SAMN15532581 | 18.52 |
| Bacteroides ovatus Y | SAMN15532582 | 2.22 |
| Bacteroides ovatus Z | SAMN15532671 | 3.1 |
| Bacteroides stercoris A | SAMN15533330 | 6.5 |
| Bacteroides stercoris B | SAMN15532522 | 5.95 |
| Bacteroides stercoris C | SAMN15532604 | 5.62 |
| Bacteroides stercoris D | SAMN15532979 | 7.33 |
| Bacteroides stercoris E | SAMN15533129 | 2.26 |
| Bacteroides thetaiotaomicron A | SAMN15532508 | 2.92 |
| Bacteroides thetaiotaomicron B | SAMN15532376 | 7.12 |
| Bacteroides thetaiotaomicron C | SAMN15533323 | 3.3 |
| Bacteroides thetaiotaomicron D | SAMN15532783 | 3.4 |
| Bacteroides thetaiotaomicron E | SAMN15532862 | 3.16 |
| Bacteroides thetaiotaomicron F | SAMN15532628 | 2.93 |
| Bacteroides thetaiotaomicron G | SAMN15533351 | 2.71 |
| Bacteroides thetaiotaomicron H | SAMN15532502 | 3.51 |
| Bacteroides thetaiotaomicron I | SAMN15532570 | 2.46 |
| Bacteroides thetaiotaomicron J | SAMN15532596 | 8.11 |
| Bacteroides thetaiotaomicron K | SAMN15532636 | 2.69 |
| Bacteroides thetaiotaomicron L | SAMN15532658 | 3.2 |
| Bacteroides thetaiotaomicron M | SAMN15532761 | 6.72 |
| Bacteroides thetaiotaomicron N | SAMN15532781 | 3.19 |
| Bacteroides thetaiotaomicron O | SAMN15532795 | 2.65 |
| Bacteroides thetaiotaomicron P | SAMN15532963 | 3.06 |
| Bacteroides thetaiotaomicron Q | SAMN15533007 | 2.31 |
| Bacteroides thetaiotaomicron R | SAMN15533013 | 3.28 |
| Bacteroides thetaiotaomicron S | SAMN15533089 | 3.27 |
| Bacteroides thetaiotaomicron T | SAMN15533093 | 3.13 |
| Bacteroides thetaiotaomicron U | SAMN15533097 | 3.22 |
| Bacteroides thetaiotaomicron V | SAMN15533127 | 7.57 |
| Bacteroides thetaiotaomicron W | SAMN15533274 | 2.94 |
| Bacteroides uniformis a | SAMN15533166 | 2.53 |
| Bacteroides uniformis A | SAMN15532497 | 1.55 |
| Bacteroides uniformis b | SAMN15533190 | 1.59 |
| Bacteroides uniformis B | SAMN15532481 | 2.6 |
| Bacteroides uniformis c | SAMN15533200 | 3.29 |
| Bacteroides uniformis C | SAMN15532535 | 2.52 |
| Bacteroides uniformis d | SAMN15533263 | 2.42 |
| Bacteroides uniformis D | SAMN15532544 | 3.12 |
| Bacteroides uniformis e | SAMN15533291 | 2.03 |
| Bacteroides uniformis E | SAMN15532603 | 3.07 |
| Bacteroides uniformis f | SAMN15533342 | 2.8 |
| Bacteroides uniformis F | SAMN15532675 | 17.59 |
| Bacteroides uniformis G | SAMN15532676 | 2.05 |
| Bacteroides uniformis g | SAMN15533345 | 2.33 |
| Bacteroides uniformis h | SAMN15533360 | 2.34 |
| Bacteroides uniformis H | SAMN15532704 | 2.6 |
| Bacteroides uniformis I | SAMN15532713 | 3.87 |
| Bacteroides uniformis J | SAMN15532744 | 4.4 |
| Bacteroides uniformis K | SAMN15532754 | 2.67 |
| Bacteroides uniformis L | SAMN15532771 | 2.49 |
| Bacteroides uniformis M | SAMN15532779 | 2.02 |
| Bacteroides uniformis N | SAMN15532843 | 2.65 |
| Bacteroides uniformis O | SAMN15532851 | 2.3 |
| Bacteroides uniformis P | SAMN15532879 | 2.2 |
| Bacteroides uniformis Q | SAMN15532886 | 2.6 |
| Bacteroides uniformis R | SAMN15532891 | 3.2 |
| Bacteroides uniformis S | SAMN15532900 | 5.53 |
| Bacteroides uniformis T | SAMN15532917 | 3.13 |
| Bacteroides uniformis U | SAMN15532932 | 2.13 |
| Bacteroides uniformis V | SAMN15532948 | 2.2 |
| Bacteroides uniformis W | SAMN15533030 | 3.54 |
| Bacteroides uniformis X | SAMN15533062 | 1.83 |
| Bacteroides uniformis Y | SAMN15533081 | 2.27 |
| Bacteroides uniformis Z | SAMN15533098 | 4.34 |
| Bacteroides vulgatus A | SAMN15532766 | 2.02 |
| Bacteroides vulgatus a | SAMN15533294 | 3.17 |
| Bacteroides vulgatus b | SAMN15533315 | 3.05 |
| Bacteroides vulgatus B | SAMN15533157 | 0.97 |
| Bacteroides vulgatus C | SAMN15532435 | 2.83 |
| Bacteroides vulgatus c | SAMN15533327 | 2.65 |
| Bacteroides vulgatus D | SAMN15532562 | 2.16 |
| Bacteroides vulgatus d | SAMN15533343 | 2.66 |
| Bacteroides vulgatus E | SAMN15532569 | 2.41 |
| Bacteroides vulgatus e | SAMN15533349 | 2.76 |
| Bacteroides vulgatus F | SAMN15532577 | 2.75 |
| Bacteroides vulgatus f | SAMN15533353 | 3 |
| Bacteroides vulgatus g | SAMN15634166 | 2.97 |
| Bacteroides vulgatus G | SAMN15532642 | 2.24 |
| Bacteroides vulgatus h | SAMN15634167 | 2.87 |
| Bacteroides vulgatus H | SAMN15532685 | 2.68 |
| Bacteroides vulgatus I | SAMN15532715 | 2.23 |
| Bacteroides vulgatus J | SAMN15532741 | 2.79 |
| Bacteroides vulgatus K | SAMN15532786 | 3.1 |
| Bacteroides vulgatus L | SAMN15532869 | 2.82 |
| Bacteroides vulgatus M | SAMN15532873 | 2.41 |
| Bacteroides vulgatus N | SAMN15532957 | 3.18 |
| Bacteroides vulgatus O | SAMN15532970 | 2.92 |
| Bacteroides vulgatus P | SAMN15532984 | 2.6 |
| Bacteroides vulgatus Q | SAMN15532985 | 2.53 |
| Bacteroides vulgatus R | SAMN15533034 | 2.75 |
| Bacteroides vulgatus S | SAMN15533073 | 2.57 |
| Bacteroides vulgatus T | SAMN15533077 | 2.35 |
| Bacteroides vulgatus U | SAMN15533099 | 3.13 |
| Bacteroides vulgatus V | SAMN15533125 | 2.56 |
| Bacteroides vulgatus W | SAMN15533189 | 2.54 |
| Bacteroides vulgatus X | SAMN15533215 | 2.5 |
| Bacteroides vulgatus Y | SAMN15533265 | 2.88 |
| Bacteroides vulgatus Z | SAMN15533276 | 1.68 |
| Bacteroides xylanisolvens A | SAMN15532721 | 17.31 |
| Bacteroides xylanisolvens B | SAMN15532738 | 1.97 |
| Bacteroides xylanisolvens C | SAMN15532817 | 3.02 |
| Bacteroides xylanisolvens D | SAMN15532925 | 3.99 |
| Bacteroides xylanisolvens E | SAMN15533273 | 2.82 |
| Bacteroides xylanisolvens F | SAMN15533357 | 2.49 |
| Barnesiella intestinihominis A | SAMN15532402 | 6.1 |
| Barnesiella intestinihominis B | SAMN15532425 | 6.11 |
| Barnesiella intestinihominis C | SAMN15532836 | 3.11 |
| Barnesiella intestinihominis D | SAMN15532954 | 3.92 |
| Barnesiella intestinihominis E | SAMN15533026 | 2.63 |
| Barnesiella intestinihominis F | SAMN15533347 | 2.65 |
| Bifidobacterium adolescentis A | SAMN15532697 | 3.05 |
| Bifidobacterium adolescentis B | SAMN15532384 | 2.56 |
| Bifidobacterium adolescentis C | SAMN15532382 | 3.26 |
| Bifidobacterium adolescentis D | SAMN15532752 | 2.94 |
| Bifidobacterium adolescentis E | SAMN15532381 | 3.18 |
| Bifidobacterium adolescentis F | SAMN15532393 | 2 |
| Bifidobacterium adolescentis G | SAMN15532437 | 2.37 |
| Bifidobacterium adolescentis H | SAMN15532549 | 4.29 |
| Bifidobacterium adolescentis I | SAMN15532600 | 1.67 |
| Bifidobacterium adolescentis J | SAMN15532606 | 3.49 |
| Bifidobacterium adolescentis K | SAMN15532607 | 2.34 |
| Bifidobacterium adolescentis L | SAMN15532611 | 2.91 |
| Bifidobacterium adolescentis M | SAMN15532612 | 1.98 |
| Bifidobacterium adolescentis N | SAMN15532365 | 2.6 |
| Bifidobacterium adolescentis O | SAMN15532746 | 3.39 |
| Bifidobacterium adolescentis P | SAMN15532789 | 3.07 |
| Bifidobacterium adolescentis Q | SAMN15532794 | 2.82 |
| Bifidobacterium adolescentis R | SAMN15533150 | 1.88 |
| Bifidobacterium adolescentis S | SAMN15533187 | 1.87 |
| Bifidobacterium adolescentis T | SAMN15533211 | 2.45 |
| Bifidobacterium adolescentis U | SAMN15533350 | 1.84 |
| Bifidobacterium bifidum A | SAMN15532824 | 2.12 |
| Bifidobacterium bifidum B | SAMN15533074 | 3.15 |
| Bifidobacterium bifidum C | SAMN15532455 | 3.1 |
| Bifidobacterium bifidum D | SAMN15532514 | 2.25 |
| Bifidobacterium bifidum E | SAMN15532543 | 2.62 |
| Bifidobacterium bifidum F | SAMN15532708 | 2.18 |
| Bifidobacterium bifidum G | SAMN15532763 | 3.12 |
| Bifidobacterium bifidum H | SAMN15532784 | 1.96 |
| Bifidobacterium longum a | SAMN15533338 | 2.28 |
| Bifidobacterium longum A | SAMN15533110 | 2.14 |
| Bifidobacterium longum b | SAMN15533352 | 1.93 |
| Bifidobacterium longum B | SAMN15532405 | 2.25 |
| Bifidobacterium longum C | SAMN15532493 | 2.17 |
| Bifidobacterium longum c | SAMN15532997 | 2.02 |
| Bifidobacterium longum D | SAMN15532877 | 2.59 |
| Bifidobacterium longum d | SAMN15533048 | 2.19 |
| Bifidobacterium longum E | SAMN15532998 | 2.38 |
| Bifidobacterium longum e | SAMN15533049 | 2.53 |
| Bifidobacterium longum f | SAMN15533139 | 2.63 |
| Bifidobacterium longum F | SAMN15532428 | 2.46 |
| Bifidobacterium longum g | SAMN15533149 | 2.18 |
| Bifidobacterium longum G | SAMN15532477 | 2.69 |
| Bifidobacterium longum H | SAMN15532480 | 2.22 |
| Bifidobacterium longum h | SAMN15533213 | 2.5 |
| Bifidobacterium longum I | SAMN15532499 | 2.31 |
| Bifidobacterium longum i | SAMN15533225 | 2.68 |
| Bifidobacterium longum j | SAMN15533229 | 2.61 |
| Bifidobacterium longum J | SAMN15532580 | 2.98 |
| Bifidobacterium longum k | SAMN15533239 | 2.37 |
| Bifidobacterium longum K | SAMN15532617 | 2.46 |
| Bifidobacterium longum L | SAMN15532648 | 5.58 |
| Bifidobacterium longum M | SAMN15532650 | 1.8 |
| Bifidobacterium longum N | SAMN15532691 | 2.25 |
| Bifidobacterium longum O | SAMN15532730 | 2.29 |
| Bifidobacterium longum P | SAMN15532765 | 2.23 |
| Bifidobacterium longum Q | SAMN15532819 | 2 |
| Bifidobacterium longum R | SAMN15532852 | 2.17 |
| Bifidobacterium longum S | SAMN15532904 | 1.79 |
| Bifidobacterium longum T | SAMN15532912 | 2.44 |
| Bifidobacterium longum U | SAMN15532921 | 2.32 |
| Bifidobacterium longum V | SAMN15532928 | 1.96 |
| Bifidobacterium longum W | SAMN15532945 | 2.93 |
| Bifidobacterium longum X | SAMN15532947 | 2.27 |
| Bifidobacterium longum Y | SAMN15532973 | 11.79 |
| Bifidobacterium longum Z | SAMN15532993 | 2.7 |
| Bifidobacterium pseudocatenulatum A | SAMN15532468 | 2.74 |
| Bifidobacterium pseudocatenulatum B | SAMN15533121 | 3.37 |
| Bifidobacterium pseudocatenulatum C | SAMN15533169 | 1.88 |
| Bifidobacterium pseudocatenulatum D | SAMN15533094 | 10.27 |
| Bifidobacterium pseudocatenulatum E | SAMN15532408 | 3.01 |
| Bifidobacterium pseudocatenulatum F | SAMN15532542 | 2.71 |
| Bifidobacterium pseudocatenulatum G | SAMN15532710 | 2.7 |
| Bifidobacterium pseudocatenulatum H | SAMN15532830 | 3.93 |
| Bifidobacterium pseudocatenulatum I | SAMN15532887 | 14.49 |
| Bifidobacterium pseudocatenulatum J | SAMN15533241 | 2.69 |
| Bifidobacterium pseudocatenulatum K | SAMN15533305 | 2.45 |
| Blautia massiliensis A | SAMN15532467 | 7.8 |
| Blautia massiliensis B | SAMN15532747 | 5.54 |
| Blautia massiliensis C | SAMN15533053 | 7.42 |
| Blautia massiliensis D | SAMN15533177 | 12.91 |
| Blautia wexlerae A | SAMN15532559 | 3.36 |
| Blautia wexlerae B | SAMN15532434 | 3.47 |
| Blautia wexlerae C | SAMN15532483 | 2.9 |
| Blautia wexlerae D | SAMN15532510 | 2.42 |
| Blautia wexlerae E | SAMN15532547 | 3.66 |
| Blautia wexlerae F | SAMN15532616 | 5.05 |
| Blautia wexlerae G | SAMN15532664 | 3.95 |
| Blautia wexlerae H | SAMN15532820 | 3.01 |
| Blautia wexlerae I | SAMN15532883 | 2.51 |
| Blautia wexlerae J | SAMN15532964 | 11.5 |
| Blautia wexlerae K | SAMN15533063 | 4.02 |
| Blautia wexlerae L | SAMN15533113 | 5.38 |
| Blautia wexlerae M | SAMN15533186 | 8.19 |
| Blautia wexlerae N | SAMN15533303 | 5.32 |
| Blautia wexlerae O | SAMN15533314 | 4.54 |
| Clostridium A | SAMN15533243 | 30.12 |
| Clostridium B | SAMN15532504 | 10.2 |
| Clostridium C | SAMN15532495 | 80.32 |
| Clostridium D | SAMN15532505 | 79.61 |
| Clostridium E | SAMN15532512 | 15.59 |
| Clostridium F | SAMN15532546 | 65.71 |
| Clostridium G | SAMN15532561 | 18.51 |
| Clostridium H | SAMN15532633 | 89.89 |
| Clostridium I | SAMN15532686 | 5.67 |
| Clostridium J | SAMN15532755 | 76.05 |
| Clostridium K | SAMN15532961 | 25.61 |
| Clostridium L | SAMN15532992 | 22.83 |
| Clostridium M | SAMN15533027 | 3.99 |
| Clostridium N | SAMN15533154 | 98.28 |
| Clostridium O | SAMN15533158 | 5.92 |
| Clostridium P | SAMN15533162 | 17.95 |
| Clostridium Q | SAMN15533230 | 4.43 |
| Clostridium R | SAMN15533232 | 79.36 |
| Clostridium S | SAMN15533313 | 2.51 |
| Collinsella aerofaciens A | SAMN15532409 | 9.05 |
| Collinsella aerofaciens B | SAMN15532411 | 18.45 |
| Collinsella aerofaciens C | SAMN15532442 | 18.91 |
| Collinsella aerofaciens D | SAMN15532573 | 11.89 |
| Collinsella aerofaciens E | SAMN15532590 | 24.84 |
| Collinsella aerofaciens F | SAMN15532593 | 7.98 |
| Collinsella aerofaciens G | SAMN15532723 | 9.13 |
| Collinsella aerofaciens H | SAMN15532742 | 6.53 |
| Collinsella aerofaciens I | SAMN15532825 | 7.85 |
| Collinsella aerofaciens J | SAMN15532936 | 11.62 |
| Collinsella aerofaciens K | SAMN15532950 | 5.76 |
| Collinsella aerofaciens L | SAMN15533076 | 5.3 |
| Collinsella aerofaciens M | SAMN15533100 | 11.35 |
| Collinsella aerofaciens N | SAMN15533103 | 22.37 |
| Collinsella aerofaciens O | SAMN15533126 | 11.08 |
| Collinsella aerofaciens P | SAMN15533171 | 2.98 |
| Collinsella aerofaciens Q | SAMN15533192 | 7.78 |
| Collinsella aerofaciens R | SAMN15533197 | 4.04 |
| Collinsella aerofaciens S | SAMN15533199 | 7.29 |
| Collinsella aerofaciens T | SAMN15533214 | 7.35 |
| Collinsella aerofaciens U | SAMN15533240 | 8.19 |
| Collinsella aerofaciens V | SAMN15533307 | 3.88 |
| Coprococcus comes A | SAMN15532605 | 2.91 |
| Coprococcus comes B | SAMN15532977 | 3.07 |
| Coprococcus comes C | SAMN15532575 | 3.09 |
| Coprococcus comes D | SAMN15532673 | 4.54 |
| Coprococcus comes E | SAMN15532792 | 3.88 |
| Coprococcus comes F | SAMN15532811 | 7.76 |
| Coprococcus comes G | SAMN15532823 | 43.5 |
| Coprococcus comes H | SAMN15532990 | 1.79 |
| Coprococcus comes I | SAMN15533028 | 4.7 |
| Coprococcus comes J | SAMN15533075 | 2.27 |
| Coprococcus comes K | SAMN15533136 | 2.62 |
| Coprococcus comes L | SAMN15533143 | 4.43 |
| Coprococcus comes M | SAMN15533210 | 3.29 |
| Coprococcus comes N | SAMN15533249 | 6.34 |
| Coprococcus comes O | SAMN15533250 | 1.93 |
| Coprococcus comes P | SAMN15533318 | 4.26 |
| Dorea longicatena A | SAMN15532943 | 3.25 |
| Dorea longicatena B | SAMN15532530 | 2.8 |
| Dorea longicatena C | SAMN15532729 | 3.95 |
| Dorea longicatena D | SAMN15532767 | 3.86 |
| Dorea longicatena E | SAMN15532803 | 4.26 |
| Dorea longicatena F | SAMN15532918 | 5.48 |
| Dorea longicatena G | SAMN15533051 | 3.49 |
| Dorea longicatena H | SAMN15533231 | 4.74 |
| Eggerthella lenta A | SAMN15532420 | 4.14 |
| Eggerthella lenta B | SAMN15532403 | 3.05 |
| Eggerthella lenta C | SAMN15532412 | 5.2 |
| Eggerthella lenta D | SAMN15532427 | 3 |
| Eggerthella lenta E | SAMN15532513 | 1.42 |
| Eggerthella lenta F | SAMN15532527 | 3.36 |
| Eggerthella lenta G | SAMN15532598 | 3.07 |
| Eggerthella lenta H | SAMN15532728 | 3.95 |
| Eggerthella lenta I | SAMN15532805 | 5.25 |
| Eggerthella lenta J | SAMN15532967 | 3.52 |
| Eggerthella lenta K | SAMN15532972 | 6.43 |
| Eggerthella lenta L | SAMN15533018 | 2.88 |
| Eggerthella lenta M | SAMN15533037 | 3.98 |
| Eggerthella lenta N | SAMN15533071 | 2.48 |
| Eggerthella lenta O | SAMN15533227 | 2.73 |
| Eggerthella lenta P | SAMN15533284 | 5.37 |
| Eggerthella lenta Q | SAMN15533319 | 13.64 |
| Escherichia coli A | SAMN15532860 | 3.03 |
| Escherichia coli a | SAMN15533009 | 3.34 |
| Escherichia coli B | SAMN15533311 | 2.01 |
| Escherichia coli b | SAMN15533032 | 3.64 |
| Escherichia coli c | SAMN15533039 | 2.95 |
| Escherichia coli C | SAMN15532419 | 3.46 |
| Escherichia coli D | SAMN15532496 | 3.21 |
| Escherichia coli d | SAMN15533172 | 3.37 |
| Escherichia coli e | SAMN15533258 | 1.66 |
| Escherichia coli E | SAMN15532507 | 2.03 |
| Escherichia coli f | SAMN15533260 | 1.94 |
| Escherichia coli F | SAMN15532521 | 8.94 |
| Escherichia coli G | SAMN15532584 | 1.73 |
| Escherichia coli g | SAMN15533344 | 1.47 |
| Escherichia coli h | SAMN15533359 | 2.41 |
| Escherichia coli H | SAMN15532614 | 1.63 |
| Escherichia coli i | SAMN15634168 | 1.56 |
| Escherichia coli I | SAMN15532619 | 2.49 |
| Escherichia coli J | SAMN15532623 | 1.22 |
| Escherichia coli K | SAMN15532638 | 3.5 |
| Escherichia coli L | SAMN15532652 | 5.73 |
| Escherichia coli M | SAMN15532660 | 2.64 |
| Escherichia coli N | SAMN15532661 | 2.81 |
| Escherichia coli O | SAMN15532698 | 3.1 |
| Escherichia coli P | SAMN15532718 | 2.41 |
| Escherichia coli Q | SAMN15532722 | 4.09 |
| Escherichia coli R | SAMN15532782 | 1.16 |
| Escherichia coli S | SAMN15532813 | 1.73 |
| Escherichia coli T | SAMN15532832 | 2.82 |
| Escherichia coli U | SAMN15532858 | 2.45 |
| Escherichia coli V | SAMN15532874 | 2.86 |
| Escherichia coli W | SAMN15532881 | 3.38 |
| Escherichia coli X | SAMN15532897 | 2.72 |
| Escherichia coli Y | SAMN15532909 | 2.51 |
| Escherichia coli Z | SAMN15532960 | 2.85 |
| Eubacterium rectale A | SAMN15532976 | 2.79 |
| Eubacterium rectale B | SAMN15532474 | 3.12 |
| Eubacterium rectale C | SAMN15532665 | 3.68 |
| Eubacterium rectale D | SAMN15532667 | 3.69 |
| Eubacterium rectale E | SAMN15532692 | 3.98 |
| Eubacterium rectale F | SAMN15532740 | 2.4 |
| Eubacterium rectale G | SAMN15532901 | 5.6 |
| Eubacterium rectale H | SAMN15533134 | 2.31 |
| Eubacterium siraeum A | SAMN15532563 | 8.85 |
| Eubacterium siraeum B | SAMN15532841 | 11.03 |
| Eubacterium siraeum C | SAMN15532845 | 6.51 |
| Eubacterium siraeum D | SAMN15533072 | 11.72 |
| Eubacterium siraeum E | SAMN15533133 | 6.28 |
| Eubacterium tenue A | SAMN15533261 | 2.31 |
| Odoribacter splanchnicus A | SAMN15533209 | 2.35 |
| Odoribacter splanchnicus B | SAMN15532613 | 1.5 |
| Odoribacter splanchnicus C | SAMN15532668 | 3.06 |
| Odoribacter splanchnicus D | SAMN15532798 | 2.62 |
| Odoribacter splanchnicus E | SAMN15532837 | 2.24 |
| Odoribacter splanchnicus F | SAMN15533012 | 2.01 |
| Odoribacter splanchnicus G | SAMN15533041 | 2.94 |
| Odoribacter splanchnicus H | SAMN15532373 | 2.74 |
| Odoribacter splanchnicus I | SAMN15533346 | 2.17 |
| Parabacteroides distasonis A | SAMN15532962 | 32.06 |
| Parabacteroides distasonis B | SAMN15532395 | 5.21 |
| Parabacteroides distasonis C | SAMN15532410 | 10.98 |
| Parabacteroides distasonis D | SAMN15532464 | 2.41 |
| Parabacteroides distasonis E | SAMN15532492 | 2.71 |
| Parabacteroides distasonis F | SAMN15532672 | 3.15 |
| Parabacteroides distasonis G | SAMN15532702 | 2.9 |
| Parabacteroides distasonis H | SAMN15532793 | 3.12 |
| Parabacteroides distasonis I | SAMN15532959 | 1.7 |
| Parabacteroides distasonis J | SAMN15532982 | 4.86 |
| Parabacteroides distasonis K | SAMN15533000 | 3.09 |
| Parabacteroides distasonis L | SAMN15533024 | 3.25 |
| Parabacteroides distasonis M | SAMN15533066 | 2.62 |
| Parabacteroides distasonis N | SAMN15533102 | 14.54 |
| Parabacteroides distasonis O | SAMN15533120 | 3.55 |
| Parabacteroides distasonis P | SAMN15533238 | 3.84 |
| Parabacteroides distasonis Q | SAMN15533251 | 5.21 |
| Parabacteroides distasonis R | SAMN15533257 | 5.18 |
| Parabacteroides distasonis S | SAMN15533333 | 5.46 |
| Parabacteroides distasonis T | SAMN15533358 | 1.56 |
| Parabacteroides johnsonii A | SAMN15532407 | 16.05 |
| Parabacteroides johnsonii B | SAMN15532635 | 16.15 |
| Parabacteroides johnsonii C | SAMN15533348 | 2.25 |
| Parabacteroides merdae A | SAMN15532955 | 3.27 |
| Parabacteroides merdae B | SAMN15532439 | 2.71 |
| Parabacteroides merdae C | SAMN15532560 | 2.5 |
| Parabacteroides merdae D | SAMN15532601 | 2.24 |
| Parabacteroides merdae E | SAMN15532615 | 9.3 |
| Parabacteroides merdae F | SAMN15532705 | 2.71 |
| Parabacteroides merdae G | SAMN15532731 | 1.86 |
| Parabacteroides merdae H | SAMN15532831 | 9.41 |
| Parabacteroides merdae I | SAMN15533003 | 2.45 |
| Parabacteroides merdae J | SAMN15533086 | 2.48 |
| Parabacteroides merdae K | SAMN15533105 | 2.38 |
| Parabacteroides merdae L | SAMN15533152 | 4.52 |
| Parabacteroides merdae M | SAMN15533221 | 2.17 |
| Roseburia faecis A | SAMN15533295 | 3.63 |
| Roseburia faecis B | SAMN15532589 | 3.92 |
| Roseburia faecis C | SAMN15532366 | 2.79 |
| Roseburia faecis D | SAMN15532815 | 3.54 |
| Roseburia faecis E | SAMN15532828 | 3.33 |
| Roseburia faecis F | SAMN15533145 | 3.9 |
| Roseburia faecis G | SAMN15533275 | 2.5 |
| Ruminococcus A | SAMN15533219 | 4.16 |
| Ruminococcus B | SAMN15532790 | 9.21 |
| Ruminococcus C | SAMN15532396 | 4.29 |
| Ruminococcus D | SAMN15532399 | 25.01 |
| Ruminococcus E | SAMN15532682 | 26.04 |
| Ruminococcus F | SAMN15532735 | 3.56 |
| Ruminococcus G | SAMN15532835 | 8.9 |
| Ruminococcus H | SAMN15532840 | 9.55 |
| Ruminococcus I | SAMN15532850 | 61.33 |
| Ruminococcus J | SAMN15532868 | 27.52 |
| Ruminococcus K | SAMN15532924 | 7.33 |
| Ruminococcus L | SAMN15532937 | 8.57 |
| Ruminococcus M | SAMN15532939 | 5.98 |
| Ruminococcus N | SAMN15533014 | 2.26 |
| Ruminococcus O | SAMN15533183 | 3.98 |
| Ruminococcus P | SAMN15533218 | 4.41 |
| Ruminococcus Q | SAMN15533252 | 3.63 |
| Ruminococcus R | SAMN15533259 | 5.29 |
| Ruminococcus torques A | SAMN15532461 | 6.84 |
| Ruminococcus torques B | SAMN15532654 | 4.52 |
| Ruminococcus torques C | SAMN15533116 | 6.05 |
| Streptococcus parasanguinis A | SAMN15532501 | 6.12 |
| Streptococcus parasanguinis B | SAMN15532487 | 7.29 |
| Streptococcus parasanguinis C | SAMN15532564 | 7.39 |
| Streptococcus parasanguinis D | SAMN15532640 | 10.35 |
| Streptococcus parasanguinis E | SAMN15533108 | 6.6 |
| Streptococcus parasanguinis F | SAMN15533119 | 8.43 |
| Streptococcus parasanguinis G | SAMN15533141 | 9.94 |
| Streptococcus parasanguinis H | SAMN15533206 | 3.49 |
| Streptococcus parasanguinis I | SAMN15533253 | 9.04 |
| Streptococcus parasanguinis J | SAMN15533302 | 9.49 |
| Streptococcus parasanguinis K | SAMN15533308 | 9.1 |
| Streptococcus pasteurianus A | SAMN15532893 | 47.64 |
| Streptococcus salivarius A | SAMN15532919 | 4.15 |
| Streptococcus salivarius B | SAMN15532459 | 8.66 |
| Streptococcus salivarius C | SAMN15532460 | 4.16 |
| Streptococcus salivarius D | SAMN15532472 | 6.64 |
| Streptococcus salivarius E | SAMN15532531 | 10.64 |
| Streptococcus salivarius F | SAMN15532532 | 7.72 |
| Streptococcus salivarius G | SAMN15532541 | 10.22 |
| Streptococcus salivarius H | SAMN15532548 | 5.07 |
| Streptococcus salivarius I | SAMN15532680 | 3.77 |
| Streptococcus salivarius J | SAMN15532745 | 3.86 |
| Streptococcus salivarius K | SAMN15532776 | 7.3 |
| Streptococcus salivarius L | SAMN15532810 | 7.2 |
| Streptococcus salivarius M | SAMN15532863 | 2.98 |
| Streptococcus salivarius N | SAMN15532864 | 16.8 |
| Streptococcus salivarius O | SAMN15533036 | 5.99 |
| Streptococcus salivarius P | SAMN15533038 | 3.99 |
| Streptococcus salivarius Q | SAMN15533060 | 3.09 |
| Streptococcus salivarius R | SAMN15533194 | 2.46 |
| Streptococcus sobrinus A | SAMN15533106 | 65.7 |

## Claims

1. A composition consisting essentially of a formulation consisting of the following bacterial strains :
| | |
|---|---|
| *Bacteroides uniformis* | MTCO1.01 |
| *Bacteroides ovatus* | MTCO1.02 |
| *Bifidobacterium longum* | MTCO1.03 |
| *Bacteroides thetaiotaomicron* | MTCO1.04 |
| *Bacteroides vulgatus* | MTCO1.05 |
| *Parabacteroides distasemis* | MTCO1.06 |
| *Bifidobacterium adolescentis* | MTCO1.07 |
| *Parabacteroides merdae* | MTCO1.08 |
| *Coprococcus comes* | MTCO1.09 |
| *Eubacterium rectale* | MTCO1.10 |
| *Bacteroides caccae* | MTCO1.11 |
| *Dorea fongicatena* | MTCO1.12 |
| *Odoribacter splanchnicus* | MTCO1.13 |
| *Bacteroides cellulosilyticus* | MTCO1.14 |
| *Bifdobacterium pseudocatenulatum* | MTCO1.15. |

2. A composition for use in treating diseases, disorders, or maladies of the human gastrointestinal tract, wherein the composition consists essentially of a formulation consisting of the following bacterial strains:
| | |
|---|---|
| *Bacteroides uniformis* | MTCO1.01 |
| *Bacteroides ovatus* | MTCO1.02 |
| *Bifidobacterium longum* | MTCO1.03 |
| *Bacteroides thetaiota*o*micron* | MTCO1.04 |
| *Bacteroides vulgatus* | MTCO1.05 |
| *Parabacteroides distasonis* | MTCO1.06 |
| *Bifidobacterium adolescentis* | MTCO1.07 |
| *Parabacteroides merdae* | MTCO1.08 |
| *Coprococcus comes* | MTCO1.09 |
| *Eubacterium rectale* | MTCO1.10 |
| *Bacteroides caccae* | MTCO1.11 |
| *Dorea fongicatena* | MTCO1.12 |
| *Odoribacter splanchnicus* | MTCO1.13 |
| *Bacteroides cellulosilyticus* | MTCO1.14 |
| *Bifdobacterium pseudocatenulatum* | MTCO1.15 |
and
wherein the diseases, disorders, or maladies of the human gastrointestinal tract are a *Clostridioides difficile* infection.

## Patentansprüche

1. Zusammensetzung, die im Wesentlichen aus einer Formulierung besteht, die aus den folgenden Bakterienstämmen besteht:
| | |
|---|---|
| *Bacteroides uniformis* | MTCO1.01 |
| *Bacteroides ovatus* | MTCO1.02 |
| *Bifidobacterium longum* | MTCO1.03 |
| *Bacteroides thetaiotaomicron* | MTCO1.04 |
| *Bacteroides vulgatus* | MTCO1.05 |
| *Parabacteroides distasonis* | MTCO1.06 |
| *Bifidobacterium adolescentis* | MTCO1.07 |
| *Parabacteroides merdae* | MTCO1.08 |
| *Coprococcus comes* | MTCO1.09 |
| *Eubacterium rectale* | MTCO1.10 |
| *Bacteroides caccae* | MTCO1.11 |
| *Dorea fongicatena* | MTCO1.12 |
| *Odoribacter splanchnicus* | MTCO1.13 |
| *Bacteroides cellulosilyticus* | MTCO1.14 |
| *Bifdobacterium pseudocate nulatum* | MTCO1.15. |

2. Zusammensetzung zur Verwendung bei der Behandlung von Krankheiten, Störungen oder Leiden des menschlichen Magen-Darm-Trakts, wobei die Zusammensetzung im Wesentlichen aus einer Formulierung besteht, die aus den folgenden Bakterienstämmen besteht:
| | |
|---|---|
| *Bacteroides uniformis* | MTCO1.01 |
| *Bacteroides ovatus* | MTCO1.02 |
| *Bifidobacterium longum* | MTCO1.03 |
| *Bacteroides thetaiotaomicron* | MTCO1.04 |
| *Bacteroides vulgatus* | MTCO1.05 |
| *Parabacteroides distasonis* | MTCO1.06 |
| *Bifidobacterium adolescentis* | MTCO1.07 |
| *Parabacteroides merdae* | MTCO1.08 |
| *Coprococcus comes* | MTCO1.09 |
| *Eubacterium rectale* | MTCO1.10 |
| *Bacteroides caccae* | MTCO1.11 |
| *Dorea fongicatena* | MTCO1.12 |
| *Odoribacter splanchnicus* | MTCO1.13 |
| *Bacteroides cellulosilyticus* | MTCO1.14 |
| *Bifdob*acterium *pseudocatenulatum* | MTCO1.15, |
und
wobei es sich bei den Krankheiten, Störungen oder Leiden des menschlichen Magen-Darm-Trakts um eine Clostridioides-difficile-Infektion handelt.

## Revendications

1. Une composition constituée essentiellement d'une formulation comprenant les souches bactériennes suivantes :
| | |
|---|---|
| *Bacteroides uniformis* | MTCO1.01 |
| *Bacteroides ovatus* | MTCO1.02 |
| *Bifidobacterium longum* | MTCO1.03 |
| *Bacteroides thetaiotaomicron* | MTCO1.04 |
| *Bacteroides vulgatus* | MTCO1.05 |
| *Parabacteroides distasonis* | MTCO1.06 |
| *Bifidobacterium adolescentis* | MTCO1.07 |
| *Parabacteroides merdae* | MTCO1.08 |
| *Coprococcus comes* | MTCO1.09 |
| *Eubacterium rectale* | MTCO1.10 |
| *Bacteroides caccae* | MTCO1.11 |
| *Dorea fongicatena* | MTCO1.12 |
| *Odoribacter splanchnicus* | MTCO1.13 |
| *Bacteroides cellulosilyticus* | MTCO1.14 |
| *Bifdobacterium pseudocate nulatum* | MTCO1.15 |

2. Une composition destinée à être utilisée dans le traitement de maladies, de troubles ou d'affections du tractus gastro-intestinal humain, la composition étant constituée essentiellement d'une formulation comprenant les souches bactériennes suivantes :
| | |
|---|---|
| *Bacteroides uniformis* | MTCO1.01 |
| *Bacteroides ovatus* | MTCO1.02 |
| *Bifidobacterium longum* | MTCO1.03 |
| *Bacteroides thetaiotaomicron* | MTCO1.04 |
| *Bacteroides vulgatus* | MTCO1.05 |
| *Parabacteroides distasonis* | MTCO1.06 |
| *Bifidobacterium adolescentis* | MTCO1.07 |
| *Parabacteroides merdae* | MTCO1.08 |
| *Coprococcus comes* | MTCO1.09 |
| *Eubacterium rectale* | MTCO1.10 |
| *Bacteroides caccae* | MTCO1.11 |
| *Dorea longicatena* | MTCO1.12 |
| *Odoribacter splanchnicus* | MTCO1.13 |
| *Bacteroides cellulosilyticus* | MTCO1.14 |
| *Bifdobacterium pseudocatenulatum* | MTCO1.15 |
les maladies, troubles ou affections du tractus gastro-intestinal humain correspondant à une infection à *Clostridioides difficile.*
